# EUROPEAN PATENT SPECIFICATION

(11) **EP 1 646 874 B1**
(45) Date of publication and mention of the grant of the patent: **03.03.2010**
(21) Application number: 04740845.5
(22) Date of filing: 09.07.2004
(51) Int. Cl.: G01N 33/68, G01N 33/574

(54) **A METHOD FOR PREDICTING THE METASTATIC POTENTIAL OF BREAST CANCER**
VERFAHREN ZUR VORHERSAGE DER METASTASEFÄHIGKEIT VON MAMMAKARZINOM
PROCEDE POUR DETERMINER LE POTENTIEL METASTATIQUE D'UN CANCER DU SEIN

(30) Priority: 11.07.2003 EP 03015584
(43) Date of publication of application: 19.04.2006
(73) Proprietor: ZEILLINGER, Robert, 2602 Blumau-Neurisshof (AT)
(72) Inventor: EVTIMOVA, Vesna, 22119 Hamburg (DE); WEIDLE, Ulrich, 80336 München (DE); ZEILLINGER, Robert, A-2602 Blumau-Neurisshof (AT)
(74) Representative: Redl, Gerda
(86) International application number: PCT/EP2004/007561
(87) International publication number: WO 2005/005989

(56) References cited:
- US-A1- 2002 146 687
- HAZAN R B ET AL: "EXOGENOUS EXPRESSION OF N-CADHERIN IN BREAST CANCER CELLS INDUCES CELL MIGRATION, INVASION AND METASTASIS" THE JOURNAL OF CELL BIOLOGY, ROCKEFELLER UNIVERSITY PRESS, US, vol. 148, no. 4, 21 February 2000 (2000-02-21), pages 779-790, XP002944580 ISSN: 0021-9525
- VOGEL W F ET AL: "DISCOIDIN DOMAIN RECEPTOR 1 TYROSINE KINASE HAS AN ESSENTIAL ROLE IN MAMMARY GLAND DEVELOPMENT" MOLECULAR AND CELLULAR BIOLOGY, AMERICAN SOCIETY FOR MICROBIOLOGY, WASHINGTON, US, vol. 21, no. 8, April 2001 (2001-04), pages 2906-2917, XP001155625 ISSN: 0270-7306
- EVTIMOVA VESNA ET AL: "Identification of breast cancer metastasis-associated genes by chip technology" ANTICANCER RESEARCH, vol. 21, no. 6A, November 2001 (2001-11), pages 3799-3806, XP009037909 ISSN: 0250-7005
- EUER NICOLE ET AL: "Identification of genes associated with metastasis of mammary carcinoma in metastatic versus non-metastatic cell lines" ANTICANCER RESEARCH, vol. 22, no. 2A, March 2002 (2002-03), pages 733-740, XP009037910 ISSN: 0250-7005
- MACKAY A ET AL: "CDNA MICROARRAY ANALYSIS OF GENES ASSOCIATED WITH ERBB2 (HER2/NEU) OVEREXPRESSION IN HUMAN MAMMARY LUMINAL EPITHELIAL CELLS" ONCOGENE, BASINGSTOKE, HANTS, GB, vol. 22, no. 17, 1 May 2003 (2003-05-01), pages 2680-2688, XP009018980 ISSN: 0950-9232
- EVTIMOVA VESNA ET AL: "Identification of genes associated with the invasive status of human mammary carcinoma cell lines by transcriptional profiling." TUMOR BIOLOGY, vol. 24, no. 4, August 2003 (2003-08), pages 189-198, XP009037917 ISSN: 1010-4283

### SEQUENCE LISTING

<110> F. Hoffmann-La Roche AG
<120> A method for predicting the metastatic potential of breast cancer
<130> 21832
<150> EP 03015584
   <151> 2003-07-11
<160> 4
<170> PatentIn version 3.2
<210> 1
   <211> 3275
   <212> DNA
   <213> Homo sapiens
<400> 1
   cggacgcgtg ggcgacccac gcgtccggcc tgtggatgta tgcctaccac cgggctcctt 60
   caccagcaaa gtggaaaaag aagcgtttca caacaaattc ttctttttgg gttggggaaa 120
   cgcagtggat tatagctctg ttttcttctt tccaaaactg tgcacccctg gatgaaacct 180
   ccatcaaggg agacctacaa gttgcctggg gttcagtgct ctagaaagtt ccaaggtttg 240
   tggcttgaat tattctaaag aagctgaaat aattgaagag aagcagaggc cagctgtttt 300
   tgaggatcct gctccacaga gaatgctctg cacccgttga tatgcctccc aggacccaga 360
   gggagactgt agcctcattt ctgtggagac ctttggctgg actctcctgg ctctcccaga 420
   gactccagtt ccaacaccat cttctgagat gatcctgatt cccagaatgc tcttggtgct 480
   gttcctgctg ctgcctatct tgagttctgc aaaagctcag gttaatccag ctatatgccg 540
   ctatcctctg ggcatgtcag gaggccagat tccagatgag gacatcacag cttccagtca 600
   gtggtcagag tccacagctg ccaaatatgg aaggctggac tcagaagaag gggatggagc 660
   ctggtgccct gagattccag tggaacctga tgacctgaag gagtttctgc agattgactt 720
   gcacaccctc cattttatca ctctggtggg gacccagggg cgccatgcag gaggtcatgg 780
   catcgagttt gcccccatgt acaagatcaa ttacagtcgg gatggcactc gctggatctc 840
   ttggcggaac cgtcatggga aacaggtgct ggatggaaat agtaacccct atgacatttt 900
   cctaaaggac ttggagccgc ccattgtagc cagatttgtc cggttcattc cagtcaccga 960
   ccactccatg aatgtgtgta tgagagtgga gctttacggc tgtgtctggc tagatggctt 1020
   ggtgtcttac aatgctccag ctgggcagca gtttgtactc cctggaggtt ccatcattta 1080
   tctgaatgat tctgtctatg atggagctgt tggatacagc atgacagaag ggctaggcca 1140
   attgaccgat ggtgtgtctg gcctggacga tttcacccag acccatgaat accacgtgtg 1200
   gcccggctat gactatgtgg gctggcggaa cgagagtgcc accaatggct acattgagat 1260
   catgtttgaa tttgaccgca tcaggaattt cactaccatg aaggtccact gcaacaacat 1320
   gtttgctaaa ggtgtgaaga tctttaagga ggtacagtgc tacttccgct ctgaagccag 1380
   tgagtgggaa cctaatgcca tttccttccc ccttgtcctg gatgacgtca accccagtgc 1440
   tcggtttgtc acggtgcctc tccaccaccg aatggccagt gccatcaagt gtcaatacca 1500
   ttttgcagat acctggatga tgttcagtga gatcaccttc caatcagatg ctgcaatgta 1560
   caacaactct gaagccctgc ccacctctcc tatggcaccc acaacctatg atccaatgct 1620
   taaagttgat gacagcaaca ctcggatcct gattggctgc ttggtggcca tcatctttat 1680
   cctcctggcc atcattgtca tcatcctctg gaggcagttc tggcagaaaa tgctggagaa 1740
   ggcttctcgg aggatgctgg atgatgaaat gacagtcagc ctttccctgc caagtgattc 1800
   tagcatgttc aacaataacc gctcctcatc acctagtgaa caagggtcca actcgactta   1860
   cgatcgcatc tttccccttc gccctgacta ccaggagcca tccaggctga tacgaaaact   1920
   cccagaattt gctccagggg aggaggagtc aggctgcagc ggtgttgtga agccagtcca 1980
   gcccagtggc cctgaggggg tgccccacta tgcagaggct gacatagtga acctccaagg 2040
   agtgacagga ggcaacacat actcagtgcc tgccgtcacc atggacctgc tctcaggaaa 2100
   agatgtggct gtggaggagt tccccaggaa actcctaact ttcaaagaga agctgggaga 2160
   aggacagttt ggggaggttc atctctgtga agtggaggga atggaaaaat tcaaagacaa 2220
   agattttgcc ctagatgtca gtgccaacca gcctgtcctg gtggctgtga aaatgctccg 2280
   agcagatgcc aacaagaatg ccaggaatga ttttcttaag gagataaaga tcatgtctcg 2340
   gctcaaggac ccaaacatca tccatctatt agctgtgtgt atcactgatg accctctctg 2400
   tatgatcact gaatacatgg agaatggaga tctcaatcag tttctttccc gccacgagcc 2460
   ccctaattct tcctccagcg atgtacgcac tgtcagttac accaatctga agtttatggc 2520
   tacccaaatt gcctctggca tgaagtacct ttcctctctt aattttgttc accgagatct 2580
   ggccacacga aactgtttag tgggtaagaa ctacacaatc aagatagctg actttggaat 2640
   gagcaggaac ctgtacagtg gtgactatta ccggatccag ggccgggcag tgctccctat 2700
   ccgctggatg tcttgggaga gtatcttgct gggcaagttc actacagcaa gtgatgtgtg   2760
   ggcctttggg gttactttgt gggagacttt caccttttgt caagaacagc cctattccca   2820
   gctgtcagat gaacaggtta ttgagaatac tggagagttc ttccgagacc aagggaggca   2880
   gacttacctc cctcaaccag ccatttgtcc tgactctgtg tataagctga tgctcagctg   2940
   ctggagaaga gatacgaaga accgtccctc attccaagaa atccaccttc tgctccttca   3000
   acaaggcgac gagtgatgct gtcagtgcct ggccatgttc ctacggctca ggtcctccct   3060
   acaagaccta ccactcaccc atgcctatgc cactccatct ggacatttaa tgaaactgag   3120
   agacagaggc ttgtttgctt tgccctcttt tcctggtcac ccccactccc tacccctgac   3180
   tcatatatac tttttttttt ttacattaaa gaactaaaaa aggaaaaaaa aaaaaaaaaa   3240
   aaaaaaaaaa aaaaaaaaaa aaaaaaaaaa aaaaa                             3275
<210> 2
   <211> 817
   <212> DNA
   <213> Homo sapiens
<400> 2
   gaaggaggcc cagacagtga gggcaggagg gagagaagag acgcagaagg agagcgagcg 60
   agagagaaag ggttctggat tggaggggag agcaagggag ggaggaaggc ggtgagagag 120
   gcgggggcct cgggagggtg aaaggaggga ggagaagggc ggggcacgga ggcccgagcg 180
   agggacaaga ctccgactcc agctctgact tttttcgcgg ctctcggctt ccactgcagc 240
   catgtcactc ctcttgctgg tggtctcagc ccttcacatc ctcattctta tactgctttt 300
   cgtggccact ttggacaagt cctggtggac tctccctggg aaagagtccc tgaatctctg 360
   gtacgactgc acgtggaaca acgacaccaa aacatgggcc tgcagtaatg tcagcgagaa 420
   tggctggctg aaggcggtgc aggtcctcat ggtgctctcc ctcattctct gctgtctctc 480
   cttcatcctg ttcatgttcc agctctacac catgcgacga ggaggtctct tctatgccac 540
   cggcctctgc cagctttgca ccagcgtggc ggtgtttact ggcgccttga tctatgccat 600
   tcacgccgag gagatcctgg agaagcaccc gcgagggggc agcttcggat actgcttcgc 660
   cctggcctgg gtggccttcc ccctcgccct ggtcagcggc atcatctaca tccacctacg 720
   gaagcgggag tgagcgcccc gcctcgctcg gctgcccccg ccccttcccg gcccccctcg 780
   ccgcgcgtcc tccaaaaaaa taaaacttta acggcgg 817
<210> 3
   <211> 3448
   <212> DNA
   <213> Homo sapiens
<400> 3
   gactgggtca tccctccaat caacttgcca gaaaactcca ggggaccttt tcctcaagag 60
   cttgtcagga tcaggtctga tagagataaa aacctttcac tgcggtacag tgtaactggg 120
   ccaggagctg accagcctcc aactggtatc ttcattctca accccatctc gggtcagctg 180
   tcggtgacaa agcccctgga tcgcgagcag atagcccggt ttcatttgag ggcacatgca 240
   gtagatatta atggaaatca agtggagaac cccattgaca ttgtcatcaa tgttattgac 300
   atgaatgaca acagacctga gttcttacac caggtttgga atgggacagt tcctgaggga 360
   tcaaagcctg gaacatatgt gatgaccgta acagcaattg atgctgacga tcccaatgcc 420
   ctcaatggga tgttgaggta cagaatcgtg tctcaggctc caagcacccc ttcacccaac 480
   atgtttacaa tcaacaatga gactggtgac atcatcacag tggcagctgg acttgatcga 540
   gaaaaagtgc aacagtatac gttaataatt caagctacag acatggaagg caatcccaca 600
   tatggccttt caaacacagc cacggccgtc atcacagtga cagatgtcaa tgacaatcct 660
   ccagagttta ctgccatgac gttttatggt gaagttcctg agaacagggt agacatcata 720
   gtagctaatc taactgtgac cgataaggat caaccccata caccagcctg gaacgcagtg 780
   tacagaatca gtggcggaga tcctactgga cggttcgcca tccagaccga cccaaacagc 840
   aacgacgggt tagtcaccgt ggtcaaacca atcgactttg aaacaaatag gatgtttgtc 900
   cttactgttg ctgcagaaaa tcaagtgcca ttagccaagg gaattcagca cccgcctcag 960
   tcaactgcaa ccgtgtctgt tacagttatt gacgtaaatg aaaaccctta ttttgccccc 1020
   aatcctaaga tcattcgcca agaagaaggg cttcatgccg gtaccatgtt gacaacattc 1080
   actgctcagg acccagatcg atatatgcag caaaatatta gatacactaa attatctgat 1140
   cctgccaatt ggctaaaaat agatcctgtg aatggacaaa taactacaat tgctgttttg 1200
   gaccgagaat caccaaatgt gaaaaacaat atatataatg ctactttcct tgcttctgac 1260
   aatggaattc ctcctatgag tggaacagga acgctgcaga tctatttact tgatattaat 1320
   gacaatgccc ctcaagtgtt acctcaagag gcagagactt gcgaaactcc agaccccaat 1380
   tcaattaata ttacagcact tgattatgac attgatccaa atgctggacc atttgctttt 1440
   gatcttcctt tatctccagt gactattaag agaaattgga ccatcactcg gcttaatggt 1500
   gattttgctc agcttaattt aaagataaaa tttcttgaag ctggtatcta tgaagttccc 1560
   atcataatca cagattcggg taatcctccc aaatcaaata tttccatcct gcgcgtgaag 1620
   gtttgccagt gtgactccaa cggggactgc acagatgtgg acaggattgt gggtgcgggg 1680
   cttggcaccg gtgccatcat tgccatcctg ctctgcatca tcatcctgct tatccttgtg 1740
   ctgatgtttg tggtatggat gaaacgccgg gataaagaac gccaggccaa acaactttta 1800
   attgatccag aagatgatgt aagagataat attttaaaat atgatgaaga aggtggagga 1860
   gaagaagacc aggactatga cttgagccag ctgcagcagc ctgacactgt ggagcctgat 1920
   gccatcaagc ctgtgggaat ccgacgaatg gatgaaagac ccatccacgc cgagccccag 1980
   tatccggtcc gatctgcagc cccacaccct ggagacattg gggacttcat taatgagggc 2040
   cttaaagcgg ctgacaatga ccccacagct ccaccatatg actccctgtt agtgtttgac 2100
   tatgaaggca gtggctccac tgctgggtcc ttgagctccc ttaattcctc aagtagtggt 2160
   ggtgagcagg actatgatta cctgaacgac tgggggccac ggttcaagaa acttgctgac 2220
   atgtatggtg gaggtgatga ctgaacttca gggtgaactt ggtttttgga caagtacaaa 2280
   caatttcaac tgatattccc aaaaagcatt cagaagctag gctttaactt tgtagtctac 2340
   tagcacagtg cctgctggag gctttggcat aggctgcaaa ccaatttggg ctcagaggga 2400
   atatcagtga tccatactgt ttggaaaaac actgagctca gttacacttg aattttacag 2460
   tacagaagca ctgggatttt atgtgccttt ttgtaccttt ttcagattgg aattagtttt 2520
   ctgtttaagg ctttaatggt actgatttct gaaacgataa gtaaaagaca aaatattttg 2580
   tggtgggagc agtaagttaa accatgatat gcttcaacac gcttttgtta cattgcattt 2640
   gcttttatta aaatacaaaa ttaaacaaac aaaaaaactc atggagcgat tttattatct 2700
   tgggggatga gaccatgaga ttggaaaatg tacattactt ctagttttag actttagttt 2760
   gttttttttt tttttcacta aaatcttaaa acttactcag ctggttgcaa ataaagggag 2820
   ttttcatatc accaatttgt agcaaaattg aattttttca taaactagaa tgttagacac 2880
   attttggtct taatccatgt acaccttttt atttctgtat ttttccactt cactgtaaaa 2940
   atagtatgtg tacataatgt tttattggca tacgtctatg gagaagtgca gaaacttcag 3000
   aacatgtgta tgtattattt ggactatgga ttcaggtttt ttgcatgttt atatctttcg 3060
   ttatggataa agtatttaca aaacagtgac atttgattca attgttgagc tgtagttaga 3120
   atactcaatt tttaattttt ttaatttttt tattttttat tttctttttg gtttggggag 3180
   ggagaaaagt tcttagcaca aatgttttac ataatttgta ccaaaaaaaa aaaaaaagga 3240
   aaggaaagaa aggggtggcc tgacactggt ggcactacta agtgtgtgtt tttttaaaaa 3300
   aaaaatggaa aaaaaaaagc ctttaaactg gagagacttc tgacaacagc tttgcctctg 3360
   tattgtgtac cagaatataa atgatacacc tctgacccca gcgttctgaa taaaatgcta 3420
   attttggata acaaaaaaag gggaattc 3448
<210> 4
   <211> 1806
   <212> DNA
   <213> Homo sapiens
<400> 4
   agggaacatc tcggggagcc tggttggaag ctgcaggctt agtctgtcgg ctgcgggtct      60
   ctgactgccc tgtggggagg gtcttgcctt aacatccctt gcatttggct gcaaagaaat     120
   ctgcttggaa gaaggggtta cgctgtttgg ccgggcagaa actccgctga gcagaacttg     180
   ccgccagaat gctcctcctg ttgctgagta tcatcgtcct ccacgtcgcg gtgctggtgc     240
   tgctgttcgt ctccacgatc gtcagccaat ggatcgtggg caatggacac gcaactgatc     300
   tctggcagaa ctgtagcacc tcttcctcag gaaatgtcca ccactgtttc tcatcatcac     360
   caaacgaatg gctgcagtct gtccaggcca ccatgatcct gtcgatcatc ttcagcattc     420
   tgtctctgtt cctgttcttc tgccaactct tcaccctcac caaggggggc aggttttaca     480
   tcactggaat cttccaaatt cttgctggtc tgtgcgtgat gagtgctgcg gccatctaca     540
   cggtgaggca cccggagtgg catctcaact cggattactc ctacggtttc gcctacatcc     600
   tggcctgggt ggccttcccc ctggcccttc tcagcggtgt catctatgtg atcttgcgga     660
   aacgcgaatg aggcgcccag acggtctgtc tgaggctctg agcgtacata gggaagggag     720
   gaagggaaac cagaaagcag acaaagaaaa aagagctagc ccaaaatccc aaactcaaac     780
   caaaccaaac agaaagcagt ggaggtgggg gttgctgttg attgaagatg tatataatat     840
   ctccggttta taaaacctat ttataacact ttttacatat atgtacatag tattgtttgc     900
   tttttatgtt gaccatcagc ctcgtgttga gccttaaaga agtagctaag gaactttaca     960
   tggtaacagt ataatccagc tcagtatttt tgttttgttt tttgtttgtt tgttttgttt    1020
   tacccagaaa taagataact ccatgtcgcc ccttcccttt catctgaaag aagatacctc    1080
   cctcccagtc cacctcattt agaaaaccaa agtgtgggta gaaaccccaa atgtccaaaa    1140
   gcccttttct ggtgggtgac ccagtgcatc caacagaaac agccgctgcc cgaacctgtg    1200
   tgaagcttta cgcgcacacg gacaaaatgc ccaaactgga gcccttgcaa aaacacggct    1260
   tgtggcattg gcatacttgc ccttacaggt ggagtatctt cgtcacacat ctaaatgaga    1320
   aatcagtgac aacaagtctt tgaaatggtg ctatggattt accattcctt attatcacta    1380
   atcatctaaa caactcactg gaaatccaat taacaatttt acaacataag atagaatgga    1440
   gacctgaata attctgtgta atataaatgg tttataactg cttttgtacc tagctaggct    1500
   gctattatta ctataatgag taaatcataa agccttcatc actcccacat tttccttacg    1560
   gtcggagcat cagaacaagc gtctagactc cttgggaccg tgagttccta gagcttggct    1620
   gggtctaggc tgttctgtgc ctccaaggac tgtctggcaa tgacttgtat tggccaccaa    1680
   ctgtagatgt atatatggtg cccttctgat gctaagactc cagacctttt gtttttgctt    1740
   tgcattttct gattttatac caactgtgtg gactaagatg cattaaaata aacatcagag    1800
   taactc                                                                1806

## Description

### Field of the Invention

A process for determining whether a tumor sample has potential for metastasis or tumor progression is provided whereby the overexpression of four genes, transmembrane receptor tyrosine kinase DDR2, transmembrane receptors PMP22 and TEMP3 and cell adhesion molecule N-cadherin in a sample is analyzed. Kits suitable for performing the invention are also provided.

### Background of the Invention

Since every eighth women in Europe and the US succumbs to breast cancer, diagnosis and treatment of this disease is a major challenge. Breast cancer is a heterogeneous type of neoplasm which contains subpopulations of cells with different angiogenic, invasive and metastatic properties. Metastasis selects for a small subpopulation of cells that preexist within the parental neoplasm. Mammary carcinoma metastases are resistant to conventional therapies. The identification of genes and their products involved in mediating an invasive phenotype is therefore of paramount importance for gaining access to validated new prognostic markers and potential targets for therapy.

Array technology facilitates the simultaneous analysis of the expression of thousands of genes simultaneously. Gene expression profiles from individual samples correlate with cancer subclasses (Golub, T.R., et al., Science 286 (1999) 531-537; Iizuka, N., Cancer Res. 62 (2002) 3939-3944), pathological status (Ramaswamy, S., et al., Nat. Genet. 33 (2003) 49-54) and clinical outcome (Wigle, D.A., et al., Cancer Res. 62 (2002) 3005-3008). Microarray analysis for breast cancer was e.g. performed by Ahr, A., et al., Lancet 359 (2002) 131-132) or Worsham, M.J., et al., Cytometry 47 (2002) 56-59).

It was an object of the present invention to provide new predictors of the outcome of breast cancer in order to allow the evaluation of the risk of metastasis in breast cancer.

### Summary of the Invention

The transcriptional profiles of four invasive and four non-invasive mammary carcinoma cell lines were derived by Affymetrix GeneChip^{®} Technology with the profile of HMEC (Human Mammary Epithelial Cells) as a baseline. It was focused on the identification of genes which are upregulated in the invasive cell lines based on the following threshold levels: fold change of two or higher in at least three or more cell lines. According to the scoring criteria 18 transmembrane receptors, 18 secreted proteins and 5 kinases were identified. Several of the genes described have already been put into context with invasion of mammary carcinoma. It was therefore focused on deregulated genes for which such an association has not been described before: transmembrane receptor tyrosine kinase DDR2 (SEQ ID NO: 1), transmembrane receptors EMP3 (SEQ ID NO: 2) and PMP22 (SEQ ID NO: 4) and and cell adhesion molecule N-cadherin (SEQ ID NO: 3). Making use of real-time PCR, consistently increased steady-state levels of mRNA's for these genes was found in an extended panel of invasive and non-invasive mammary cancer cell lines.

In an embodiment of the invention, a method of determining whether or not a human mammary cancer cell containing patient sample has potential for tumor progression or metastasis is provided, the method comprising comparing:
a) the level of expression of marker genes in the patient sample, and
b) the normal level of expression of said marker genes in a sample from a control subject not afflicted with mammary cancer or with the normal level of expression of said marker genes in a sample containing cells from a human mammary epithelial cell line,
wherein said marker genes comprise the group of marker genes consisting of the genes with a nucleic acid sequence according to SEQ ID NO: 1 (DDR2), 2 (EMP3), 3 (N-cadherin) and 4 (PMP22) and a significant difference between the level of expression of said marker genes in the patient sample and the normal level of one or several of said marker genes in the sample from a control subject not afflicted with mammary cancer or in the sample containing cells from a human mammary epithelial cell line is an indication that the patient sample has potential for tumor progression or metastasis.

A significant difference is an at least two fold difference between the level of expression of said marker genes in the patient sample and the normal level of expression of the same marker gene in the sample from the control subject or in the sample containing cells from a human mammary epithelial cell line. The method can be performed in protein or nucleic acid assays.

As used herein and the claims, each of the following terms has the meaning associated with it in this section.

According to the invention, a "solid phase" may be controlled pore glass (CPG), polystyrene or silica gel as used for oligonucleotide synthesis.

By "array" is meant an arrangement of addressable locations on a device (see, e.g., US 5,143,854; US 6,022,963; US 6,156,501; WO 90/15070; WO 92/10092). The locations can be arranged in two dimensional arrays, three dimensional arrays, or other matrix formats. The number of locations can range from several to at least hundreds of thousands. Most importantly, each location represents a totally independent reaction site. Each location carries a nucleic acid as e.g. an "oligomeric compound", which can serve as a binding partner for a second nucleic acid, in particular a target nucleic acid. Methods for the manufacturing thereof are described in EP-A 0 476 014 and Hoheisel, J. D., TIBTECH 15 (1997) 465-469; WO 89/10977; WO 89/11548; US 5,202,231; US 5,002,867; WO 93/17126. Further development has provided methods for making very large arrays of oligonucleotide probes in very small areas (US 5,143,854; WO 90/15070; WO 92/10092). Microfabricated arrays of large numbers of oligonucleotide probes, called "DNA chips" offer great promise for a wide variety of applications (see, e.g., US 6,156,501 and US 6,022,963).

In the context of this invention, "hybridization" shall mean hydrogen bonding, which may be Watson-Crick, Hoogsteen or reversed Hoogsteen hydrogen bonding, between complementary nucleotides. For example, adenine and thymine are complementary nucleobases which pair through the formation of hydrogen bonds.

"Complementary," as used herein, also refers to sequence complementarity between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity such that stable and specific binding occurs between the oligonucleotide and the DNA or RNA target. It is understood that an oligonucleotide need not be 100% complementary to its target DNA sequence to be specifically hybridizable. An oligonucleotide is specifically hybridizable when binding of the oligonucleotide to the target DNA or RNA molecule interferes with the normal function of the target DNA or RNA, and there is a sufficient degree of complementarity to avoid non-specific binding of the oligonucleotide to non-target sequences under conditions in which specific binding is desired, i.e. under physiological conditions in the case of in vivo assays or therapeutic treatment, or in the case of in vitro assays, under conditions in which the assays are performed.

The articles "a" and "an" are used herein to refer to one or to more than one (i.e. s to at least one) of the grammatical object of the article. By way of example, "an element" means one element or more than one element.

The term "marker gene" is meant to include a gene which is useful according to this invention for the evaluation of the potential of metastasis or progression of breast cancer. It could be also termed breast cancer marker gene.

The term "marker polynucleotide" is meant to include nucleotide transcript (hnRNA or mRNA) encoded by a breast cancer marker gene according to the invention, or cDNA derived from the nucleotide transcript, or a segment of said 1 o transcript or cDNA.

The term "marker protein" is meant to include protein or polypeptide encoded by a breast cancer marker gene, preferably a marker gene according to the invention, or a polypeptide or protein fragment comprising said marker protein.

The term "gene product" is meant to include marker polynucleotide and marker 5 protein encoded by the referenced gene.

As used herein the term "polynucleotide" is synonymous with "nucleic acid."

The terms "breast cancer" and "mammary cancer" are used interchangeably throughout the application.

The term "progression" of cancer shall include the event of recurrence or metastasis which is regarded to be a more specific event during progression of cancer. The progression of breast cancer is "inhibited" if recurrence or metastasis of the cancer is reduced, slowed, delayed, or prevented.

Further a polynucleotide "corresponds to" another (a first) polynucleotide if it is related to the first polynucleotide by any of the following relationships: the second polynucleotide comprises the first polynucleotide and the second polynucleotide encodes a gene product; the second polynucleotide is the complement of the first polynucleotide and, the second polynucleotide is 5' or 3' to the first polynucleotide in cDNA, RNA, genomic DNA, or fragment of any of these polynucleotides. For example, a second polynucleotide may be a fragment of a gene that includes the first and second; polynucleotides. The first and second polynucleotides are related in that they are components of the gene coding for a gene product, such as a protein or antibody.

However, it is not necessary that the second polynucleotide comprises or overlaps with the first polynucleotide to be encompassed within the definition of "corresponding to" as used herein. For example, the first polynucleotide may be a segment of a 3' untranslated region of the second polynucleotide. The first and second polynucleotide may be fragments of a gene coding for a gene product. The second polynucleotide may be an exon of the gene while the first polynucleotide may be an intron of the gene.

The term "probe" refers to any molecule which is capable of selectively binding to a specifically intended target molecule, for example a marker gene of the invention. Probes can either be synthesized by one skilled in the art, or derived from appropriate biological preparations. For purposes of detection of the target molecule, probes may be specifically designed to be labeled, as described herein. Examples of molecules that can be utilized as probes include, but are not limited to, proteins, antibodies, organic monomers, RNA, DNA, and cDNA.

A "breast-associated" body fluid is a fluid which, when in the body of a patient, contacts or passes through breast cells or into which cells, nucleic acids or proteins shed from breast cells are capable of passing. Exemplary breast-associated body fluids include blood fluids, lymph, and cystic fluid.

The "normal" level of expression of a marker gene is the level of expression of the marker gene in breast cells or breast-associated body fluids of a subject, e.g a human, not afflicted with breast cancer.

"Over-expression" and "under-expression" of a marker gene refer to expression of the marker gene of a patient at a greater or lesser level, respectively, than normal level of expression of the marker gene (e.g. at least two-fold greater or lesser level).

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue-specific manner.

A "constitutive" promoter is a nucleotide sequence which, when operably linked with a polynucleotide which encodes or specifies a gene product, causes the gene product to be produced in a living human cell under most or all physiological conditions of the cell. An "inducible" promoter is a nucleotide sequence which, when operably linked with a polynucleotide which encodes or specifies a gene product, causes the gene product to be produced in a living human cell substantially only when an inducer which corresponds to the promoter is present in the cell.

A "tissue-specific" promoter is a nucleotide sequence which, when operably linked with a polynucleotide which encodes or specifies a gene product, causes the gene product to be produced in a living human cell substantially only if the cell is a cell of the tissue type corresponding to the promoter.

A "transcribed polynucleotide" is a polynucleotide (e.g an RNA, a cDNA, or an analog of one of an RNA or cDNA) which is complementary to or homologous with all or a portion of a mature RNA made by transcription of a gene, such as any of the marker genes of the invention, and normal post-transcriptional processing (e.g. splicing), if any, of the transcript.

"Complementary" refers to the broad concept of sequence complementarily between regions of two nucleic acid strands or between two regions of the same nucleic acid strand. It is known that an adenine residue of a first nucleic acid region is capable of fortning specific hydrogen bonds ("base pairing") with a residue of a second nucleic 5 acid region which is antiparallel to the first region if the residue is thymine or uracil.

Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. Preferably, the first region comprises a first portion and the second region comprises a second portion, whereby when the first and second portions are arranged in an antiparallel fashion, at least about 50%, and preferably at least about 75%, at least about 90%, or at least about 95% of the 5 nucleotide residues of the first portion are capable of base pairing with nucleotide residues in the second portion. More preferably, all nucleotide residues of the first portion are capable of base pairing with nucleotide residues in the second portion.

"Homologous" as used herein, refers to nucleotide sequence similarity between two regions of the same nucleic acid strand or between regions of two different nucleic acid strands. Homology between two regions is expressed in terms of the proportion of nucleotide residue positions of the two regions that are occupied by the same nucleotide residue. By way of example, a region having the nucleotide sequence 5`-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

A nucleic acid or protein is "fixed" to a substrate if it is covalently or non-covalently associated with the substrate such that the substrate can be rinsed with a fluid (e.g standard saline citrate, pH 7.4) without a substantial fraction of the nucleic acid or protein dissociating from the substrate.

As used herein, a "naturally-occurring" nucleic acid molecule refers to an RNA or DNA molecule having a nucleotide sequence that occurs in nature.

Expression of a marker gene in a patient is "significantly" altered from the level of expression of the marker gene in a control subject if the level of expression of the marker gene in a sample from the patient differs from the level in a sample from the control subject by an amount greater than the standard error of the assay employed to assess expression, and preferably at least twice, and more preferably three, four, five or ten times that amount. Expression of a marker gene in a patient is "significantly" higher than the level of expression of the marker gene in a control subject if the level of 5 expression of the marker gene in a sample from the patient is greater than the level in a sample from the control subject by an amount greater than the standard error of the assay employed to assess expression, and preferably at least twice, and more preferably three, four, five or ten times that amount. Alternately, expression of the marker gene in the patient can be considered "significantly" lower than the level of expression in a control subject if the level of expression in a sample from the patient is lower than the level in a sample from the control subject by an amount greater than the standard error of the assay employed to assess expression, and preferably at least twice, and more preferably three, four, five or ten times that amount.

A "kit" is any manufacture (e.g a package or container) comprising at least one reagent, e.g a probe, for specifically detecting a marker gene or peptide of the invention. The manufacture is preferably promoted, distributed, or sold as a unit for performing the methods of the present invention.

Various specific aspects of the invention are described in further detail in the following subsections.

### Detailed description of the invention

In an embodiment of the invention, a method of determining whether or not a human mammary cancer cell containing patient sample has potential for tumor progression or metastasis is provided, the method comprising comparing:
a) the level of expression of marker genes in the patient sample, and
b) the normal level of expression of said marker genes in a sample from a control subject not afflicted with mammary cancer or the normal level of expression of said marker genes in a sample containing cells from a human mammary epithelial cell line,
wherein said marker genes comprise the group of marker genes consisting of the genes with a nucleic acid sequence according to SEQ ID NO: 1 (DDR2), 2 (EMP3), 3 (N-cadherin) and 4 (PMP22)
and a significant difference between the level of expression of said marker genes in the patient sample and the normal level of said marker genes in the sample from a control subject not afflicted with mammary cancer or in the sample containing cells from a human mammary epithelial cell line is an indication that the patient sample has potential for tumor progression or metastasis.

Preferably said marker genes consists of the group of marker genes with a nucleic acid sequence according to SEQ ID NO: 1 (DDR2), 2 (EMP3), 3 (N-cadherin) and 4 (PMP22). This is also preferred for all other embodiments of the invention.

In another embodiment of the invention, a method of determining whether or not a human mammary cancer cell containing patient sample has potential for tumor progression or metastasis is provided, the method comprising comparing:
a) the level of expression of marker genes in the patient sample, and
b) the normal level of expression of said marker genes in a sample from a control subject not afflicted with mammary cancer or the normal level of expression of of one or several of said marker genes in a sample containing cells from a human mammary epithelial cell line,
wherein said marker genes comprise the group consisting of the genes with a nucleic acid sequence according to SEQ ID NO: 1, 2, 3 and 4,
and a significant difference between the level of expression of said marker genes in the patient sample and the normal level of said marker genes in the sample from a control subject not afflicted with mammary cancer or in the sample containing cells from a human mammary epithelial cell line is an indication that the patient sample has potential for tumor progression or metastasis.

A significant difference is an at least two fold difference between the level of expression of said marker genes in the patient sample and the normal level of expression of the same marker genes in the sample from the control subject or in the sample containing cells from a human mammary epithelial cell line. The method can be performed in protein or nucleic acid assays.

In another preferred embodiment of the invention, a kit for assessing whether a patient has a risk of progression of breast cancer, wherein the kit comprises reagents for assessing expression of one or several marker genes and said marker genes are comprising the group consisting of the genes with a nucleic acid sequence according to (SEQ ID NO: 1 (DDR2), 2 (EMP3), 3 (N-cadherin) and 4 (PMP22) is used. The kit comprises a plurality of reagents, each of which is capable of binding specially with a protein or nucleic acid encoded by a marker gene of the invention. Suitable reagents for binding with a protein encoded by a marker gene of the invention include antibodies, antibody derivatives, antibody fragments, and the like. Additional reagents for specifically binding with a protein encoded by a marker gene include any natural ligands of the protein and derivatives of such ligands. Suitable reagents for binding with a nucleic acid encoded by a marker gene (e.g. an hnRNA, a spliced mRNA, a cDNA corresponding to the mRNA, or the like) include complementary nucleic acids. For example, the nucleic acid reagents may include oligonucleotides (labeled or non-labeled) fixed to a substrate, labeled 5 oligonucleotides not bound with a substrate, pairs of PCR primers, molecular beacon probes, and the like. The kit of the invention may optionally comprise additional components useful for performing the methods of the invention. By way of example, the kit may comprise; fluids (e.g SSC buffer) suitable for binding an antibody with a protein with which it specifically binds or, for annealing complementary nucleic acids one or more sample compartments, instructional material which describes performance of a method of the invention, a sample of control cells, a sample of breast cancer cells, and the like.

In the methods according to the invention, a significant difference comprises an at least 2 fold difference between the level of expression of said marker genes in the patient sample and the normal level of expression of the same marker genes. More preferably, the significant difference comprises an at least 5 fold difference between the level of expression of said marker genes in the patient sample and the normal level of expression of the same marker genes.

According to the present invention, the level of expression of a marker gene in a sample can be determined, for example, by detecting the level in the sample of:
- a protein encoded by the marker gene, or a polypeptide or a fragment comprising the protein (e.g using a reagent, such as an antibody, an antibody derivative, which binds specifically with the protein or a fragment thereof);
- a metabolite which is produced directly by catalysis or indirectly by; the protein encoded by the marker gene; and/or a polynucleotide (e.g. an mRNA, hnRNA, cDNA) produced by or derived from the expression of the marker gene or a fragment of the polynucleotide (e. g by contacting polynucleotides obtained or derived from the sample with a substrate having affixed thereto a nucleic acid comprising the marker gene sequence or a portion of such sequence).

A significantly altered, preferably increased, level of expression in the patient sample of the marker genes relative to those marker genes' expression levels in samples from subjects serving as a control, is an indication that the patient has a higher risk of progression of breast cancer.

Preferred in vivo techniques for detection of a protein encoded by marker genes of the invention include introducing into a subject an antibody that specifically binds the protein, or a polypeptide or protein fragment comprising the protein. In certain embodiments, the antibody can be labeled with a radioactive molecule whose presence and location in a subject can be detected by standard imaging techniques.

Expression of a marker gene of the invention may be assessed by any of a wide variety of well known methods for detecting expression of a transcribed molecule or protein. Non-limiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods. Such method may also include physical methods such as liquid and gas chromatography, mass spectroscopy, and nuclear magnetic resonance.

In a preferred embodiment, expression of a marker gene is assessed using an antibody (e.g a radio-labeled, chromophore-labeled, fluorophore- labeled, or enzyme-labeled antibody), an antibody derivative (e.g. an antibody conjugated with a substrate or with the protein or ligand of a protein-ligand pair {e.g biotin-skeptavidin}), or an antibody fragment (e.g a single-chain antibody, an isolated antibody hypervariable domain, etc.) which binds specifically with a protein encoded by the marker gene or a polypeptide or a protein fragment comprising the protein, wherein the protein may have undergone none, all or a portion of its normal post-translational modification and/or proteolysis during the course of its secretion or release from preferably colorectal cells, cancerous or otherwise.

In another preferred embodiment, expression of a marker gene is assessed by preparing mRNA/cDNA (i. e. a transcribed polynucleotide) from cells in a patient sample, and by hybridizing the mRNA/cDNA with a reference polynucleotide which comprises the marker gene sequence or its complement, or a fragment of said sequence or complement. cDNA can, optionally, be amplified using any of a variety of polymerase chain reaction methods prior to hybridization with the reference polynucleotide. Expression of one or more marker genes can likewise be detected using quantitative PCR to assess the level of RNA transcripts encoded by the marker gene(s).

In a related embodiment, a mixture of transcribed polynucleotides obtained from the sample is contacted with a substrate having fixed thereto a polynucleotide complementary to or homologous with at least a portion (e.g at least 7, 10, 15, 20, 25, 30, 40, 50, 100, 500, or more nucleotide residues) of a RNA transcript encoded by a marker gene of the invention. If polynucleotides complementary to or homologous with a RNA transcript encoded by the marker gene of the invention are differentially detectable on the substrate (e.g detectable using radioactivity, different chromophores or fluorophores), are fixed to different selected positions, then the levels of expression of a plurality of marker genes can be assessed simultaneously using a single substrate (e.g a "gene chip" microarray of polynucleotides fixed at selected positions). When a method of assessing marker gene expression is used which involves hybridization of one nucleic acid with another, it is preferred that the hybridization be performed under stringent hybridization conditions.

Because the compositions, kits, and methods of the invention rely on detection of a difference in expression levels of one or more marker genes of the invention, it is preferable that the level of expression of the marker gene is greater, preferably significantly greater, than the minimum detection limit of the method used.

The marker genes of the invention may also be used in a panel of marker genes, for example. It is well known that certain types of genes, such as oncogenes, tumor suppressor genes, growth factor-like genes, protease- like genes, and protein kinase-like genes are often involved with development of cancers of various types. Known oncogenes and tumor suppressor genes include, for example, abl, abr, akt2, apc, bc12a, bc12,C, bc13, bcr, brcal, brca2, cbl, ccudl, cdc42, cdk4, crk- II, csprlfins, dbl. ccc, dpc41smad4, e-cad, e2fflrbap, e lerbb-1, elkl, elk3, eph, erg, ets1, ets2, fer, fgrlsrc2, flillergb2, fos, fpslfes,fi al, fia2, fyn, kick, trek, her21erkb- 21nen, 5 her31erbb- 3, her41erbb-4, hrasl, hst2, hstil, igf p2, ink4a, ink4b, int21f f3, jun, junb,; jund, kip2, kit, kras2a, kras2b, Ick, Iyn, mas, mclx, mcc, mdm2, met, mlkl, mmpl O. mos, msh2, msh3, msh6, myb, myba, mybb, n yc, mycll, mycn, nfl, nf2, nme2, eras, pS3, pdgfb, phb, piml, pmsl, pms2, ptc, pten, rafl, rapla, rbl, rel, ret, rosl, ski, srcl, tall, t fbr2, tgfb3, tf br3, thral, thrb, tiaml, timp3, tjpl, tpS3, trk, vav, vhf, vil2, wafl, wntl, 10 wnt2, wtl, and yesl (Hesketh, In: The Oncogene and Tumour Suppressor Gene Facts Book, 2nd ed., Academic Press, 1997; Fishel, R., et al., Science 266 (1994) 1403-1405). Known growth factors include platelet-derived growth factor alpha, platelet derived growth factor beta (simian sarcoma viral {v-sis) oncogene homolog), thrombopoletin (myeloproliferative leukemia virus oncogene ligand, megakaryocyte 5 growth and development factor), erythropoletin, B cell growth factor, macrophage stimulating factor 1 (hepatocyte growth factor-like protein), hepatocyte growth factor (hepapoietin A), insulin-like growth factor 1 (somatomedia C), hepatoma- derived growth factor, amphiregulin (schwannoma-derived growth factor), bone morphogenetic; proteins 1, 2, 3, 3 beta, and 4, bone morphogenetic protein 7 (osteogenic protein 1), bone morphogenetic protein 8 (osteogenic protein 2), connective tissue growth factor, connective tissue activation peptide 3, epidermal growth factor (EGF), teratocarcinoma derived growth factor 1, endothelin, endothelin 2, endothelin 3, stromal cell-derived factor 1, vascular endothelial growth factor (VEGF), VEGF-B, VEGF-C, placental growth factor (vascular endothelial growth factor-related protein), transforming growth 25 factor alpha, transforming growth factor beta 1 and its precursors, transforming growth factor beta 2 and its precursors, fibroblast growth factor 1 (acidic), fibroblast growth factor 2 (basic), fibroblast growth factor 5 and its precursors, fibroblast growth factor 6 and its precursors, fibroblast growth factor 7 (keratinocyte growth factor), fibroblast growth factor 8 (androgen-induced), fibroblast growth factor 9 (glia- activating factor), pleiotrophin (heparin binding growth factor 8, neurite growth-promoting factor 1), brain-derived neurotrophic factor, and recombinant glial growth factor 2. Known proteases include interleukin- 1 beta convertase and its precursors, Mch6 and its precursors, Mch2 isoform alpha, Mch4, Cpp32 isoform alpha, Lice2 gamma cysteine protease, Ich-1 S. Ich- 1 L, Ich-2 and its precursors, TY protease, matrix 35 metalloproteinase 1 (interstitial collagenase), matrix metalloproteinase 2 (gelatinase A, 72kD gelatinase, 72kD type IV collagenase), matrix metalloproteinase 7 (matrilysin), matrix metalloproteinase 8 (neutrophil collagenase), matrix metalloproteinase 12 (macrophage elastase), matrix metalloproteinase 13 (collagenase 3), metallopeptidase 1, cysteine-rich metalloprotease (disintegrin) and its precursors, subtilisin-like protease Pc8 and its precursors, chymotrypsin, snake venom-like protease, cathepsin 1, cathepsin D (lysosomal aspartyl protease), skomelysin, aminopeptidase N. plasminogen, tissue 5 plasminogen activator, plasminogen activator inhibitor type II, and urokinase-type plasminogen activator.

The methods of the present invention may be practiced using marker genes of the invention in combination with one or more known marker genes. It will be appreciated that the methods and kits of the present invention may also include known cancer marker genes including known marker genes.

The embodiments will be described in more detail below.

### I. Predictive Medicine

The present invention pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the present invention relates to diagnostic assays for determining the level of expression of polypeptides or nucleic acids encoded by one or more marker genes of the invention, in order to determine whether an individual is at risk of metastasis or progression of breast cancer. Such assays can be used for prognostic or predictive purposes to thereby prophylactically treat an individual prior to the progression of the cancer.

Yet another aspect of the invention pertains to monitoring the influence of agents on the expression or activity of a marker gene of the invention in clinical trials. These and other agents are described in further detail in the following sections.

### A. Diagnostic Assays

In a preferred embodiment of the invention, a method of determining whether or not a human mammary cancer cell containing patient sample has potential for tumor progression or metastasis is provided, the method comprising comparing:
a) the level of expression of marker genes in the patient sample, and
b) the normal level of expression of said marker genes in a sample from a control subject not afflicted with mammary cancer or with the normal level of expression of said marker genes in a sample containing cells from a human mammary epithelial cell line,
wherein said marker genes comprise the group of marker genes consisting of the genes with a nucleic acid sequence according to SEQ ID NO: 1 (DDR2), 2 (EMP3), 3 (N-cadherin) and 4 (PMP22),
and a significant difference between the level of expression of said marker genes in the patient sample and the normal level of said marker genes in the sample from a control subject not afflicted with mammary cancer or in the sample containing cells from a human mammary epithelial cell line is an indication that the patient sample has potential for tumor progression or metastasis.

The sample is preferably a breast tissue sample, a breast-associated fluid or even urine.

One preferred embodiment of the invention is related to the detection of the proteins corresponding to the marker genes. Therefore, in an embodiment of the invention, a method according to the invention is provided, wherein the level of expression of said marker genes in the sample is assessed by detecting the presence in the sample of a protein encoded by each of said marker genes or a polypeptide or protein fragment comprising said protein. Preferably, the presence of said protein, polypeptide or protein fragment is detected using a reagent which specifically binds with said protein, polypeptide or protein fragment. The reagent is preferably selected from the group consisting of an antibody, an antibody derivative, and an antibody fragment.

Another preferred embodiment of the invention is related to the detection of the nucleic acid molecules corresponding to the marker genes. The method of claim according to the invention, wherein the level of expression of said marker genes in the sample is assessed by detecting the presence in the sample of a transcribed polynucleotide encoded by each of said marker genes or a portion of said transcribed polynucleotide. The transcribed polynucleotide is preferably a cDNA, mRNA or hnRNA. Preferably, the step of detecting further comprises amplifying the transcribed polynucleotide. More preferably, the level of expression of said marker genes in the samples is assessed by detecting the presence in the samples of a transcribed polynucleotide which anneals with each of said marker genes or anneals with a portion of said transcribed polynucleotide, under stringent hybridization conditions.

The preferred embodiments will be described in more detail below:

An exemplary method for detecting the presence or absence of a polypeptide or nucleic acid encoded by a marker gene of the invention in a biological sample involves obtaining a biological sample (e.g. a biopsy of breast tissue or a lump) from a test subject and contacting the biological sample with a compound or an agent capable of detecting the polypeptide or nucleic acid (e.g. mRNA, genomic DNA, or cDNA). The detection methods of the invention can thus be used to detect mRNA, protein, cDNA, or genomic DNA, for example, in a biological sample in vitro as well as in viva. For example, in vitro techniques for detection of mRNA include Northern hybridizations and in situ hybridizations. In vitro techniques for detection of a polypeptide encoded by a marker gene of the invention include enzyme linked immunosorbent assays (ELISAs), Western blots, immunoprecipitations, immunohistochemistry and immunofluorescence.

In vitro techniques for detection of genomic DNA include Southern hybridizations.

A general principle of such diagnostic and prognostic assays involves preparing a sample or reaction mixture that may contain a protein or nucleotide encoded by a marker gene, and a probe, under appropriate conditions and for a time sufficient to allow the protein or nucleotide and probe to interact and bind, thus forming a complex that can be removed and/or detected in the reaction mixture. These assays can be conducted in a variety of ways.

For example, one method to conduct such an assay would involve anchoring the protein or nucleotide on the one hand or probe on the other onto a solid phase supports also referred to as a substrate, and detecting complexes comprising the target marker gene or protein and the probe anchored on the solid phase at the end of the reaction. In one embodiment of such a method, a sample from a subject, which is to be assayed for presence and/or concentration of the proteins or nucleotides encoded by the marker genes, can be anchored onto a carrier or solid phase support. In another embodiment, the reverse situation is possible, in which the probe can be anchored to a solid phase and a sample from a subject can be allowed to react as an unanchored component of the assay.

There are many established methods for anchoring assay components to a solid phase. These include, without limitation, the protein or nucleotide encoded by the marker gene or probe molecules which are immobilized through conjugation of biotin and streptavidin. Such biotinylated assay components can be prepared from biotin-NHS (N-hydroxy-succinimide) using techniques known in the art (e.g. biotinylation kit, Pierce Chemicals, Rockford, IL), and immobilized in the wells of streptavidin- coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker gene protein or nucleotide or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non immobilized component is added to the solid phase upon which the second component is anchored. After the reaction is complete, uncomplexed components may be removed (e.g., by washing) under conditions such that any complexes formed will remain immobilized upon the solid phase. The detection of complexes comprising the marker protein or nucleotide sequence and the probe anchored to the solid phase can be accomplished in a number of methods outlined herein. In a preferred embodiment, the probe, when it is the unanchored assay component, can be labeled for the purpose of detection and readout of the assay, either directly or indirectly, with detectable labels discussed herein and which are well-known to one skilled in the art.

It is also possible to directly detect complexes comprising a marker protein or nucleotide sequence and the probe without further manipulation or labeling of either component (the marker protein or nucleotide or the probe), for example by utilizing the technique of fluorescence energy transfer (see, for example, U.S. Patent No. 5,631,169; U.S. Patent No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal.

A PET binding event can be conveniently measured through standard fluorometric detection means well known in the art (e.g. using a fluorimeter). In another embodiment, determination of the ability of a probe to recognize a protein or nucleotide encoded by a marker gene can be accomplished without labeling either assay component (probe or marker gene) by utilizing a technology such as real time Biomolecular Interaction Analysis (BIA) (see, e.g., Sjolander, S. and Urbaniczky, C., Anal. Chem. 63 (1991) 2338-2345; and Szabo, A., et al., Curr. Opin. Struct. Biol. 5 (1995) 699-705). As used herein, "BIA" or "surface plasmon resonance" is a technology for studying biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable which can be used as an indication of real-time reactions between biological molecules.

Alternatively, in another embodiment, analogous diagnostic and prognostic assays can be conducted with the marker protein or nucleotide and the probe as solutes in a liquid phase. In such an assay, complexes comprising the marker protein or nucleotide and the probe are separated from uncomplexed components by any of a number of standard techniques, including but not limited to: differential centrifugation, chromatography, electrophoresis and immunoprecipitation. In differential centrifugation, such complexes may be separated from uncomplexed assay components through a series of centrifugal steps, due to the different sedimentation equilibria of complexes based on their different sizes and densities (see, for example, Rivas, G., and Minton, A.P., Trends Biochem Sci. 18 (1993) 284-287). Standard chromatographic techniques may also be utilized to separate such complexes from uncomplexed components. For example, gel f ltration chromatography separates molecules based on size, and through the utilization of an appropriate gel filtration resin in a column format, for example, the relatively larger complexes may be separated from the relatively smaller uncomplexed components. Similarly, the different charge properties of such complexes as compared to the uncomplexed components may be exploited to; differentiate the complexes from uncomplexed components, for example through the utilization of ion-exchange chromatography resins. Such resins and chromatographic techniques are well known to one skilled in the art (see, e.g., Heegaard, N.H., J. Mol. Recognit. 11 (1998) 141-148; Hage, D.S., and Tweed, S.A., J. Chromatogr. B. Biomed. Sci. Appl. 699 (1997) 499-525). Gel electrophoresis may also be employed to separate such complexes from unbound components (see, e.g., Ausubel et al., ed., Current Protocols in Molecular Biology, John Wiley & Sons, New York, 1987-1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In a particular embodiment, the level of mRNA encoded by a marker gene can be determined both by in situ and by in vitro formats in a biological sample using methods known in the art. The term "biological sample" is intended to include tissues, cells, biological fluids and isolates thereof, isolated from a subject, as well as tissues, cells and fluids present within a subject. Many expression detection methods use isolated RNA.

For in vitro methods, any RNA isolation technique that does not select against the isolation of mRNA can be utilized for the purification of RNA from breast cells (see, e.g., Ausubel et al., supra). Additionally, large numbers of tissue samples can readily be processed using techniques well known to those of skill in the art, such as, for example, the single-step RNA isolation process of Chomczynski (1989, U.S. Patent No. 4,843,155).

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA encoded by a marker gene of the present invention. Other suitable probes for use in the diagnostic assays of the invention are described herein. Hybridization of a mRNA with the probe indicates that the marker gene in question is expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the a marker gene of the present invention.

An alternative method for determining the level of mRNA encoded by a marker gene of the present invention in a sample involves the process of nucleic acid amplification, e.g., by rtPCR (the experimental embodiment set forth in U.S. Patent No. 4,683,202), ligase chain reaction (Barany, F., Proc. Natl. Acad. Sci. USA 88 (1991) 189-193), self sustained sequence replication (Guatelli, J.C., et al., Proc. Natl. Acad. Sci. USA 87 (1990) 1874-1878), transcriptional amplification system (Kwoh, D.Y., et al., Proc. Natl. Acad. Sci. USA 86 (1989) 1173-1177), Q-Beta Replicase (Lizard et al., Bio/Technology 6 (1988) 1197), rolling circle replication (U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For in situ methods, mRNA does not need to be isolated from the breast cells prior to detection. In such methods, a cell or tissue sample is prepared/ processed using known histological methods. The sample is then immobilized on a support, typically a glass slide, and then contacted with a probe that can hybridize to mRNA encoded by the marker gene.

As an alternative to making determinations based on the absolute expression level of the marker gene, determinations may be based on the normalized expression level of the marker gene. Expression levels are normalized by correcting the absolute expression level of a marker gene by comparing its expression to the expression of a gene that is not a marker gene, e.g. a housekeeping gene that is constitutively expressed. Suitable genes for normalization include housekeeping genes such as the actin gene, or epithelial cell-specific genes. This normalization allows the comparison of the expression level in one sample, e.g., a patient sample, to another sample, e.g. a non breast cancer sample, or between samples from different sources.

Alternatively, the expression level can be provided as a relative expression level.

To determine a relative expression level of a marker gene, the level of expression of the marker gene or marker genes is determined for 10 or more samples of normal versus cancer cell isolates, preferably 50 or more samples, and prior to the determination of the expression level for the sample in question. The mean expression level of each of the genes assayed in the larger number of samples is determined and this is used as a baseline expression level for the marker gene. The expression level of the marker gene determined for the test sample (absolute level of expression) is then divided by the mean expression value obtained for that marker gene. This provides a relative expression level.

Preferably, the samples used in the baseline determination will be from breast cancer or from non-breast cancer cells of breast tissue. The choice of the cell source is dependent on the use of the relative expression level. Using expression found in normal tissues as a mean expression score aids in validating whether the marker gene assayed is breast specific (versus normal cells). In addition, as more data is accumulated, the mean expression value can be revised, providing improved relative expression values based on accumulated data. Expression data from breast cells provides a means for grading the severity of the breast cancer state.

In another embodiment of the present invention, a polypeptide encoded by a marker gene is detected. A preferred agent for detecting a polypeptide of the invention is an antibody capable of binding to a polypeptide encoded by a marker gene of the invention, preferably an antibody with a detectable label. Antibodies can be polyclonal, or more preferably, monoclonal. An intact antibody, or a fragment thereof (e.g. Fab or F(ab') 2) can be used. The term "labeled", with regard to the probe or antibody, is intended to encompass direct labeling of the probe or antibody by coupling (i. e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

Proteins from breast cells can be isolated using techniques that are well known to those of skill in the art. The protein isolation methods employed can, for example, be such as those described in Harlow and Lane, Antibodies: Laboratory Manual, Cold Spring Harbor Laboratory Press, Cold Spring Harbor, New York, 1988. A variety of formats can be employed to determine whether a sample contains a protein that binds to a given antibody. Examples of such formats include, but are not limited to, enzyme immunoassay (EIA), radioimmunoassay (RIA), Western blot analysis, immunohistochemistry and enzyme linked immunoabsorbant assay (ELISA).

A skilled artisan can readily adapt known protein/antibody detection methods for use in determining whether breast cells express a marker gene of the present invention.

In one format, antibodies, or antibody fragments, can be used in methods such as Western blots, immunohistochemistry or immunofluorescence techniques to detect the expressed proteins. In such uses, it is generally preferable to immobilize either the antibody, proteins, or cells containing proteins, on a solid support. Well-known supports or carriers include glass, polystyrene, polypropylene, polyethylene, dextran, nylon, amylases, natural and modified celluloses, polyacrylamides, gabbros, and magnetite.

One skilled in the art will know many other suitable carriers for binding antibody or antigen, and will be able to adapt such support for use with the present invention. For example, protein isolated from breast cells can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In another embodiment of the invention, a format for the use in the LightCycler® instrument is provided as described in US 6,174,670. These formats apply the fluorescent resonance energy transfer technology (see, for example, US Patent Nos. 4,996,143; 5,565,322; 5,849,489; and 6,162,603) and are based on the fact that when a donor and a corresponding acceptor fluorescent label are positioned within a certain distance of each other, energy transfer takes place between the two fluorescent labels that can be visualized or otherwise detected and/or quantitated. As used herein, two probes, each containing a fluorescent label, whereby the probes hybridize to the a marker gene according to the invention being the target nucleic acid, can hybridize to an amplification product at particular positions determined by the complementarity of the probes to the target nucleic acid. Upon hybridization of the probes to the amplification product at the appropriate positions, a FRET signal is generated. Fluorescent analysis can be carried out using, for example, a photon counting epifluorescent microscope system (containing the appropriate dichroic mirror and filters for monitoring fluorescent emission at the particular range), a photon counting photomultiplier system, or a fluorometer. Excitation to initiate energy transfer can be carried out with an argon ion laser, a high intensity mercury (Hg) arc lamp, a fiber optic light source, or other high intensity light source appropriately filtered for excitation in the desired range. As used herein with respect to donor and corresponding acceptor fluorescent labels, "corresponding" refers to an acceptor fluorescent label having an excitation spectrum that overlaps the emission spectrum of the donor fluorescent label. Accordingly, efficient non-radiative energy transfer can be produced there between. The preferred fluorescent label is fluorescein as the donor fluorescent label, whereby the acceptor fluorescent label is rhodamine, however, preferred is a cyanine dye, preferably Cy5 as described in US 6,174,670.

Therefore, in an embodiment of the invention, a method for detecting the presence or absence or amount of the target nucleic acid, which is a marker gene according to the invention in a sample is provided, comprising the steps of:
performing at least one cycling step, wherein a cycling step comprises an amplifying step and a hybridizing step, wherein said amplifying step comprises contacting said sample with primers to produce an amplification product if target nucleic acid is present in said sample, wherein said hybridizing step comprises contacting said sample with a pair of probes, wherein the members of said pair of probes hybridize to said amplification product within no more than five nucleotides of each other, wherein a first probe of said pair of probes is labeled with a donor fluorescent label and wherein a second probe of said pair of probes is labeled with a corresponding acceptor fluorescent label,
   and detecting the presence or absence of fluorescence resonance energy transfer between said donor fluorescent label of said first probe and said acceptor fluorescent label of said second probe, wherein the presence of FRET is indicative of the presence of the target nucleic acid in the sample, and wherein the absence of FRET is indicative of the absence of the target nucleic acid in the sample. Then the amount of target nucleic acid in the sample containing cells from a patient should be compared to the amount of target nucleic acid in a sample from an object not afflicted with breast cancer or in a sample containing cells from a human mammary epithelial cell line (HMEC).

In another preferred embodiment of the invention, a method for detecting a target nucleic acid, i.e. a marker gene according to the invention, in a sample is provided, comprising the steps of amplifying the target nucleic acid by polymerase chain reaction in the presence of two nucleic acid probes that hybridize to adjacent regions of the target nucleic acid, one of said probes being labeled with an acceptor fluorescent label and the other probe labeled with a donor fluorescent label of a fluorescence energy transfer pair such that upon hybridization of the two probes with the target nucleic acid, the donor and acceptor fluorescent labels are within 25 nucleotides of one another, said polymerase chain reaction comprising the steps of adding a thermostable polymerase, nucleotides and primers for the target nucleic acid to the sample and thermally cycling the sample between at least a denaturation temperature and an elongation temperature; exciting the biological sample with light at a wavelength absorbed by the donor fluorescent label and detecting fluorescent emission from the fluorescence energy transfer pair. Then the amount of target nucleic acid in the sample containing cells from a patient should be compared to the amount of target nucleic acid in a sample from an object not afflicted with breast cancer or in a sample containing cells from a human mammary epithelial cell line (HMEC).

In another preferred embodiment of the invention, a method for the detection of a target nucleic acid, i.e. a marker gene according to the invention, in a sample is provided comprising the steps of amplifying the target nucleic acid by polymerase chain reaction in the presence of two nucleic acid probes, that hybridize to adjacent regions of the nucleic acid, one of said probes being labeled with an acceptor fluorescent label and the other probe labeled with a donor fluorescent label of a fluorescence energy transfer pair such that upon hybridization of the two probes with the target nucleic acid, the donor and acceptor fluorescent labels are within 25 nucleotides of one another, said polymerase chain reaction comprising the steps of adding a thermostable polymerase, nucleotides and primers for the target nucleic acid to the sample and thermally cycling the sample between at least a denaturation temperature and an elongation temperature; exciting the sample with light at a wavelength absorbed by the donor label and monitoring temperature dependent fluorescence from the fluorescence energy transfer pair. Then the amount of target nucleic acid in the sample containing cells from a patient should be compared to the amount of target nucleic acid in a sample from an object not afflicted with breast cancer or in a sample containing cells from a human mammary epithelial cell line (HMEC).

The detection of DNA amplification products can also be done in other "homogeneous" assay system. A "homogeneous" assay system comprises reporter molecules or labels which generate a signal while the target sequence is amplified. Another example for a "homogeneous" assay system is the TaqMan® system that has been detailed in US 5,210,015, US 5,804,375 and US 5,487,972. Briefly, the method is based on a double-labelled probe and the 5'-3' exonuclease activity of Taq DNA polymerase. The probe is complementary to the target sequence to be amplified by the PCR process and is located between the two PCR primers during each polymerisation cycle step. The probe has two fluorescent labels attached to it. One is a reporter dye, such as 6-carboxyfluorescein (FAM), which has its emission spectra quenched by energy transfer due to the spatial proximity of a second fluorescent dye, 6-carboxy-tetramethyl-rhodamine (TAMRA). In the course of each amplification cycle, the Taq DNA polymerase in the process of elongating a primed DNA strand displaces and degrades the annealed probe, the latter due to the intrinsic 5'-3' exonuclease activity of the polymerase. The mechanism also frees the reporter dye from the quenching activity of TAMRA. As a consequence, the fluorescent activity increases with an increase in cleavage of the probe, which is proportional to the amount of PCR product formed. Accordingly, amplified target sequence is measured detecting the intensity of released fluorescence label.

Therefore, in another embodiment of the invention a method for the detection of a target nucleic acid, which is a marker gene according to the invention, in a sample is provided comprising the steps of
(a) providing a sample suspected to contain the target nucleic acid,
(b) providing an oligonucleotide, which is essentially complementary to a part or all of the target nucleic acid,
(c) optionally amplifying the target nucleic acid with a template-dependent DNA polymerase and primers,
(d) contacting the sample with the olignucleotide under conditions for binding the oligomeric compound to the target nucleic acid,
(e) determining the binding product or the degree of hybridization between the target nucleic acid and the olignucleotide as a measure of the presence, absence or amount of the target nucleic acid.

Preferably in step (d) of the method, the degree of hybridization is determined by the quantity of the first or second fluorescent label that is released from the oligonucleotide hybridized to the target nucleic acid by exonuclease hydrolysis by the template-dependent DNA polymerase. Therefor, preferably, the olignucleotide comprises two labels, preferably two fluorescent labels. Then the amount of target nucleic acid in the sample containing cells from a patient should be compared to the amount of target nucleic acid in a sample from an object not afflicted with breast cancer or in a sample containing cells from a human mammary epithelial cell line (HMEC).

In a very preferred embodiment of the invention related in more detail to the TaqMan® assay format, a method for the detection of a marker gene according to the invention, being the target nucleic acid, in a sample is provided comprising the steps of
(a) contacting a sample comprising single-stranded nucleic acids with a first oligonucleotide containing a sequence complementary to a region of the target nucleic acid and a secong oligonucleotide containing a first and a second fluorescent label, and whereby said first oligonucleotide contains a sequence complementary to a second region of the same target nucleic acid sequence strand, but not including the nucleic acid sequence defined by the second oligonucleotide, to create a mixture of duplexes during hybridization conditions, wherein the duplexes comprise the target nucleic acid annealed to the first and second oligonucleotide such that the 3' end of the first oligonucleotide is upstream of the 5' end of the oligomeric compound,
(b) maintaining the mixture of step (a) having a 5' to 3' nuclease activity under conditions sufficient to permit the 5' to 3' nuclease activity of the polymerase to cleave the annealed, oligomeric compound and release labelled fragments; and
(c) detecting and/or measuring the release of labelled fragments.

Then the amount of target nucleic acid in the sample containing cells from a patient should be compared to the amount of target nucleic acid in a sample from an object not afflicted with breast cancer or in a sample containing cells from a human mammary epithelial cell line (HMEC).

The invention also encompasses kits for detecting the presence of a polypeptide or nucleic acid encoded by marker genes of the invention in a biological sample (e.g a breast-associated body fluid). Such kits can be used to determine if a subject is suffering from or is at increased risk of metastasizing breast cancer. For example, the kit can comprise a labeled compound or agent capable of detecting a polypeptide or an mRNA encoding a polypeptide encoded by the marker genes of the invention in a biological sample and means for determining the amount of the polypeptide or mRNA in the sample (e.g. an antibody which binds the polypeptide or an oligonucleotide probe which binds to DNA or mRNA encoding the polypeptide). Kits can also include instructions for interpreting the results obtained using the kit.

For antibody-based kits, the kit can comprise, for example: (1) a first antibody (e.g. attached to a solid support) which binds to a polypeptide corresponding to a marker gene of the invention; and, optionally, (2) a second, different antibody which binds to either the polypeptide or the first antibody and is conjugated to a detectable label. For oligonucleotide-based kits, the kit can comprise, for example: (1) an oligonucleotide, e.g., a detectably labeled oligonucleotide, which hybridizes to a nucleic acid sequence encoding a polypeptide encoded by a marker gene of the invention or (2) a pair of primers useful for amplifying a nucleic acid molecule encoded by a marker gene of the invention. The kit can also comprise, e.g, a buffering agent, a preservative, or a protein stabilizing agent. The kit can further comprise components necessary for detecting the detectable label (e.g. an enzyme or a substrate). The kit can also contain a control sample or a series of control samples which can be assayed and compared to the test sample. Each component of the kit can be enclosed within an individual container and all of the various containers can be within a single package, along with instructions for interpreting the results of the assays performed using the kit.

Therefore, in another embodiment of the invention, a kit is provided for assessing whether a patient has a risk for metastasis of breast cancer, the kit comprising reagents for assessing expression of marker genes, wherein said marker genes comprise the group of marker genes consisting of the genes with a nucleic acid sequence according to SEQ ID NO: 1 (DDR2), 2 (EMP3), 3 (N-cadherin) and 4 (PMP22).

### II. Arrays

The invention is also related to an array comprising the nucleotide sequence of a marker gene or of the marker genes of the present invention. The array can be used to assay expression of one or more genes, including the marker gene, in the array. In one embodiment, the array can be used to assay gene expression in a tissue to ascertain tissue specificity of genes in the array. In this manner, several thousand genes can be simultaneously assayed for expression. This allows a profile to be developed showing a battery of genes specifically expressed in one or more tissues.

In addition to such qualitative determination, the invention allows the quantitation of marker gene expression. Thus, not only tissue specificity, but also the level of expression of a battery of genes in the tissue is ascertainable. Thus, marker genes can be grouped on the basis of their tissue expression per se and level of expression in that tissue. This is useful, for example, in ascertaining the relationship of gene expression between or among tissues. Thus, one tissue can be perturbed and the effect on marker gene expression in a second tissue can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more marker genes in the array. This can occur in various biological contexts, as disclosed herein, for example in development and differentiation of breast cancer, tumor progression, progression of other diseases, in vitro processes, such a cellular transformation and senescence, autonomic neural and neurological processes, such as, for example, pain and appetite, and cognitive functions, such as learning or memory.

The array is also useful for ascertaining the effect of the expression of a marker gene on the expression of other genes in the same cell or in different cells. This provides, for example, for a selection of alternate molecular targets for therapeutic intervention if the ultimate or downstream target cannot be regulated. The array is also useful for ascertaining differential expression patterns of one or more marker genes in normal and abnormal cells. This provides a battery of marker genes that could serve as a molecular target for diagnosis or therapeutic intervention.

### Isolated Nucleic Acid Molecules

The following paragraph is generally directed to the nucleic acid molecules used in the present invention and how to obtain them:

One aspect of the invention pertains to isolated nucleic acid molecules that correspond to a marker gene of the invention. Such nucleic acid molecules comprise sequences of RNA transcripts encoded by the marker gene or portions of such transcripts. Isolated nucleic acids of the invention also include nucleic acid molecules sufficient for use as hybridization probes to identify of RNA transcripts encoded by the marker gene or portions of such transcripts, and fragments of such nucleic acid molecules, e.g., those suitable for use as PCR primers for the amplification or mutation of nucleic acid molecules. As used herein, the term "nucleic acid molecule" is intended to include DNA molecules (e.g. cDNA or genomic DNA) and RNA molecules (e.g., mRNA) and analogs of the DNA or RNA generated using nucleotide analogs. The nucleic acid molecule can be single-stranded or double-stranded, but preferably is double- stranded DNA.

The invention also encompasses polynucleotides which differ from that of the polynucleotides described herein, but which produce the same phenotypic effect, such as an allelic variant. These altered, but phenotypically equivalent polynucleotides are referred to as "equivalent nucleic acids." This invention also encompasses polynucleotides characterized by changes in non-coding regions that do not alter the polypeptide produced therefrom when compared to the polynucleotide herein. This invention further encompasses polynucleotides, which hybridize to the polynucleotides of the subject invention under conditions of moderate or high stringency. Alternatively, the polynucleotides are at least 85%, or at least 90%, or more preferably, greater or equal to 95% identical as determined by a sequence alignment program when run under default parameters.

An "isolated" nucleic acid molecule is one which is separated from other nucleic acid molecules which are present in the natural source of the nucleic acid molecule. Preferably, an "isolated" nucleic acid molecule comprises a protein-coding sequence and is free of sequences which naturally flank the coding sequence in the genomic DNA of the organism from which the nucleic acid is derived. For example, in various embodiments, the isolated nucleic acid molecule can contain less than about 5 kB 4 kB, 3 kB, 2 kB, 1 kB, 0.5 kB or 0.1 kB of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, e.g., a nucleotide transcript encoded by a marker gene according to the invention, can be isolated using standard molecular biology techniques. Nucleic acid molecule of the present invention also encompass the marker genes of the invention, which can be isolated using standard hybridization and cloning techniques (e.g. as described in Sambrook et al., Molecular Cloning: A Laboratory Manual, 2nd ed., Cold Spring Harbor Laboratory Press, Cold Spring Harbor, NY, 1989).

A process for identifying a larger fragment or the full-length coding sequence of a marker gene of the present invention is thus also provided. Any conventional recombinant DNA techniques applicable for isolating polynucleotides may be employed. One such method involves the 5'-RACE-PCR technique, in which the poly A mRNA that contains the coding sequence of particular interest is first reverse transcribed with a 3'- primer comprising a sequence disclosed herein. The newly synthesized cDNA skand is then tagged with an anchor primer with a known sequence, which preferably contains a convenient cloning restriction site attached at the Bend.

The tagged cDNA is then amplified with the 3'-primer (or a nested primer sharing sequence homology to the internal sequences of the coding region) and the 5'-anchor primer. The amplification may be conducted under conditions of various levels, of stringency to optimize the amplification specificity. 5'-RACE-PCR can be readily performed using commercial kits (available from, e.g. BRL Life Technologies Inc., Clontech) according to the manufacturer's instructions.

Isolating the complete coding sequence of a gene can also be carried out in a hybridization assay using a suitable probe. The probe preferably comprises at least nucleotides, and more preferably exhibits sequence homology to the polynucleotides of the marker genes of the present invention. Other high throughput screens for cDNAs, such as those involving gene chip technology, can also be employed in obtaining the complete cDNA sequence. In addition, databases exist that reduce the complexity of ESTs by assembling contiguous EST sequences into tentative genes. For example, TIGR has assembled human ESTs into a database called THC for tentative human consensus sequences. The THC database allows for a more definitive assignment compared to ESTs alone.

Software programs exist (TIGR assembler and TIGEM EST assembly machine and contig assembly program (see Huang, X., 1996, Genomes 33:21-23)) that allow for assembling ESTs into contiguous sequences from any organism.

Alternatively, RNA from a sample preparation is used to construct cDNA library in the ZAP Express vector following the procedure described in Velculescu, V.E., et al., Science 270 (1995) 484-487. The ZAP Express cDNA synthesis kit (Stratagene) is used accordingly to the manufacturer's protocol. Plates containing 250 to 2000 plaques are hybridized as described in Ruppert, J.M., et al., Mol. Cell. Bio. 8 (1988) 3104-3113 to oligonucleotide probes with the same conditions previously described for standard probes except that the hybridization temperature is reduced to a room temperature. Washes are performed in 6X standard-saline-citrate 0.1% SDS for 30 minutes at room temperature. The probes are labeled with 32P-ATP trough use of T4 polynucleotide kinase.

A partial cDNA (3' fragment) can be isolated by 3' directed PCR reaction. This procedure is a modification of the protocol described in Polyak, K., et al., Nature 389 (1997) 300-305. Briefly, the procedure uses SAGE tags in PCR reaction such that the resultant PCR product contains the SAGE tag of interest as well as additional cDNA, the length of which is defined by the position of the tag with respect to the 3' end of the cDNA.

The cDNA product derived from such a transcript driven PCR reaction can be used for many applications.

RNA from a source to express the cDNA corresponding to a given tag is first converted to double-stranded cDNA using any standard cDNA protocol. Similar conditions used to generate cDNA for SAGE library construction can be employed except that a modified oligo-dT primer is used to derive the first strand synthesis. For example, the oligonucleotide of composition 5'- TCC GGC GCG COG TTT TCC CAG TCA CGA(30)-3', contains a poly-T stretch at the 3' end for hybridization and priming from poly-A tails, an M13 priming site for use in subsequent PCR steps, a 5' Biotin label (B) for capture to strepavidin-coated magnetic beads, and an AscI restriction endonuclease site for releasing the cDNA from the strepavidin-coated magnetic beads.

Theoretically, any sufficiently-sized DNA region capable of hybridizing to a PCR primer can be used as well as any other 8 base pair recognizing endonuclease. 30 cDNA constructed utilizing this or similar modified oligo-dT primer is then processed as described in U.S. Patent No. 5,695,937 up until adapter ligation where only one adapter is ligated to the cDNA pool. After adapter ligation, the cDNA is released from the streptavidin-coated magnetic beads and is then used as a template for cDNA amplification.

Various PCR protocols can be employed using PCR priming sites within the 3' modified oligo-dT primer and the SAGE tag. The SAGE tag-derived PCR primer employed can be of varying length dictated by 5' extension of the tag into the adaptor sequence. cDNA products are now available for a variety of applications.

This technique can be further modified by: (1) altering the length and/or content of the modified oligo-dT primer; (2) ligating adaptors other than that previously employed within the SAGE protocol; (3) performing PCR from template retained on the streptavidin-coated magnetic beads; and (4) priming first strand cDNA synthesis with non-oligo-dT based primers.

Gene trapper technology can also be used. The reagents and manufacturer's instructions for this technology are commercially available from Life Technologies, Inc., Gaithsburg, Maryland. Briefly, a complex population of single-stranded phagemid DNA containing directional cDNA inserts is enriched for the target sequence by hybridization in solution to a biotinylated oligonucleotide probe complementary to the target sequence. The hybrids are captured on streptavidin-coated paramagnetic beads.

A magnet retrieves the paramagnetic heads from the solution, leaving nonhybridized single-stranded DNAs behind. Subsequently, the captured single-stranded DNA target is released from the biotinylated oligonucleotide. After release, the cDNA clone is further enriched by using a nonbiotinylated target oligonucleotide to specifically prime conversion of the single-stranded DNA. Following transformation and plating, typically 20% to 100% of the clones represent the cDNA clone of interest. To identify the desired cDNA clone, the clones may be screened by colony hybridization using the 32P-labeled oligonucleotide, or alternatively by DNA sequencing and alignment of all sequences obtained from numerous clones to determine a consensus sequence.

A nucleic acid molecule of the invention can be amplified using cDNA, mRNA, or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to all or a portion of a nucleic acid molecule of the invention can be prepared by standard synthetic techniques, e.g., using an automated DNA synthesizer. In another preferred embodiment, an isolated nucleic acid molecule of the invention comprises a nucleotide sequence of a RNA transcript encoded by a marker gene of the invention or a complement of said sequence. A nucleic acid molecule which is complementary to a given nucleotide sequence is one which is sufficiently complementary to the given nucleotide sequence that it can hybridize to the given nucleotide sequence thereby forming a stable duplex.

Moreover, a nucleic acid molecule of the invention can comprise only a portion of the nucleotide sequence (RNA or cDNA) of a RNA transcript encoded by a marker gene of the invention or a complement of said sequence. Such nucleic acids can be used, for example, as a probe or primer. The probe/primer typically is used as one or more substantially purified oligonucleotides. The oligonucleotide typically comprises a region of nucleotide sequence that hybridizes under stringent conditions to at least about; 7, preferably about 15, more preferably about 25, 50, 75, 100, 125, 150, 175, 200, 250, 300, 350, or 400 or more consecutive nucleotides of a nucleic acid of the invention.

Probes based on the sequence of a nucleic acid molecule of the invention can be used to detect transcripts or genomic sequences of one or more marker genes of the invention. The probe comprises a label group attached thereto, e.g., a radioisotope, a fluorescent compound, an enzyme, or an enzyme co-factor. Such probes can be used as parts of a diagnostic test kit for identifying cells or tissues which mix-express the protein, such as by measuring levels of a nucleic acid molecule encoding the protein in a sample of cells from a subject, e.g., detecting mRNA levels or determining whether a gene encoding the protein has been mutated or deleted.

The invention further encompasses nucleic acid molecules that differ, due to degeneracy of the genetic code, from the nucleotide sequence of nucleic acids encoding a protein which corresponds to a marker gene of the invention, and thus encode the same protein.

In addition to the nucleotide sequences described in the GenBank and IMAGE; Consortium database records described herein, it will be appreciated by those skilled in the art that DNA sequence polymorphisms that lead to changes in the amino acid sequence can exist within a population (e.g., the human population). Such genetic polymorphisms can exist among individuals within a population due to natural allelic variation. An allele is one of a group of genes which occur alternatively at a; given genetic locus. In addition, it will be appreciated that DNA polymorphisms that affect RNA expression levels can also exist that may affect the overall expression level of that gene (e.g. by affecting regulation or degradation). As used herein, the phrase "allelic variant" refers to a nucleotide sequence which occurs at a given locus or to a polypeptide encoded by the nucleotide sequence.

As used herein, the terms "gene" and "recombinant gene" refer to nucleic acid molecules comprising an open reading frame encoding a polypeptide by a marker gene of the invention. Such natural allelic variations can typically result in 0. 1-0.5% variance in the nucleotide sequence of a given gene. Alternative alleles can be identified by sequencing the gene of interest in a number of different individuals.

This can be readily carried out by using hybridization probes to identify the same genetic locus in a variety of individuals. Any and all such nucleotide variations and resulting amino acid polymorphisms or variations that are the result of natural allelic variation and that do not alter the functional activity are intended to be within the scope of the invention.

In another embodiment, an isolated nucleic acid molecule of the invention is at least 7, 15, 20, 25, 30, 40, 60, 80, 100, 150, 200, 250, 30D, 350, 400, 450, 550, 650, 700, 800, 900, 1000, 1200, 1400, 1600, 1800, 2000, 2200, 2400, 2600, 2800, 3000, 3500, 4000, 4500, or more nucleotides in length and hybridizes under stringent conditions to a RNA transcript of a marker gene of the invention or a portion of said transcript or a cDNA corresponding to said transcript or portion thereof. As used herein, the term "hybridizes under stringent conditions" is intended to describe conditions for hybridization and washing under which nucleotide sequences at least 75% (80%, 85%, preferably 90%) identical to each other typically remain hybridized to each other. Such stringent conditions are known to those skilled in the art and can be found in sections 6.3.1 -6.3.6 of Current Protocols in Molecular Biology, John Wiley & Sons, N.Y., 1989. A preferred, non-limiting example of stringent hybridization conditions for annealing two single-stranded DNA each of which is at least about 100 bases in length and/or for annealing a single-stranded DNA and a single-stranded RNA each of which is at least about 100 bases in length, are hybridization in 6X sodium chloride/sodium citrate (SSC) at about 45°C, followed by one or more washes in 0.2X SSC, 0.1% SDS at 50-65°C. Further preferred hybridization conditions are taught in Lockhart, D.J., et al., Nat. Biotechnol. 14 (1996) 1675-1680; Breslauer, K.J., et al., Proc. Natl. Acad. Sci. USA 83 (1986) 3746-3750; van Ness, J., and Chen, L., Nucleic Acids Res. 19 (1991) 5143-5151; McGraw, R.A., et al., BioTechniques 8 (1990) 674-678; and Milner, N., et al., Nat. Biotechnol. 15 (1997) 537-541, all expressly incorporated by reference. In addition to naturally-occurring allelic variants of a nucleic acid molecule of the invention that can exist in the population, the skilled artisan will further appreciate that sequence changes can be introduced by mutation thereby leading to changes in the amino acid sequence of the encoded protein, without altering the biological activity of the protein encoded thereby. For example, one can make nucleotide substitutions leading to amino acid substitutions at "non-essential" amino acid residues. A "non essential" amino acid residue is a residue that can be altered from the wild-type sequence without altering the biological activity, whereas an "essential" amino acid residue is required for biological activity. For example, amino acid residues that are not conserved or only semi-conserved among homologs of various species may be non essential for activity and thus would be likely targets for alteration. Alternatively, amino acid residues that are conserved among the homologs of various species (e.g. murine and human) may be essential for activity and thus would not be likely targets for alteration. Accordingly, another aspect of the invention pertains to nucleic acid molecules encoding a polypeptide of the invention that contain changes in amino acid residues that are not essential for activity. Such polypeptides differ in amino acid sequence from the; naturally-occurring proteins encoded by the marker genes of the invention, yet retain biological activity. In one embodiment, such a protein has an amino acid sequence that is at least about 40% identical, 50%, 60%, 70%, 80%, 90%, 95%, or 98% identical to the amino acid sequence of one of the proteins encoded by the marker genes of the invention. An isolated nucleic acid molecule encoding a variant protein can be created by introducing one or more nucleotide substitutions, additions or deletions into the nucleotide sequence of nucleic acids of the invention, such that one or more amino acid residue substitutions, additions, or deletions are introduced into the encoded protein.

Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been deemed in the art. These families include amino acids with basic side chains (e.g. lysine, arginine, histidine), acidic side chains (e.g. aspartic acid, glutamic acid), uncharged polar side chains (e.g. glycine, asparagine, glutamine, serine, threonine, tyrosine, cysteine), non-polar side chains (e.g. alanine, valine, leucine, isoleucine, proline, phenylalanine, methionine, tryptophan), beta- branched side chains (e.g. threonine, valine, isoleucine) and aromatic side chains (e.g. tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed recombinantly and the activity of the protein can be determined.

The present invention encompasses antisense nucleic acid molecules, i. e., molecules which are complementary to a sense nucleic acid of the invention, e.g. complementary to the coding strand of a double-stranded cDNA molecule corresponding to a marker gene of the invention or complementary to an mRNA sequence corresponding to a marker gene of the invention. Accordingly, an antisense nucleic acid of the invention can hydrogen bond to (i. e. anneal with) a sense nucleic acid of the invention. The antisense nucleic acid can be complementary to an entire coding strand, or to only a portion thereof, e.g. all or part of the protein coding region (or open reading frame). An antisense nucleic acid molecule can also be antisense to all or part of a non- coding region of the coding strand of a nucleotide sequence encoding a polypeptide of the invention. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45, or 50 or more nucleotides in length. An antisense nucleic acid of the invention can be constructed using chemical synthesis and enzymatic ligation reactions using procedures known in the art. For example, an antisense nucleic acid (e.g. an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g. phosphorothioate derivatives and acridine substituted nucleotides can be used. Examples of modified nucleotides which can be used to generate the antisense nucleic acid include 5-fluorouracil, 5-bromouracil, 5 chlorouracil, 5-iodouracil, hypoxanthine, xanthine, 4-acetylcytosine, 5 (carboxyhydroxylmethyl) uracil, 5- carboxymethylaminomethyl-2-thiouridine, 5 carboxymethylaminomethyluracil, dihydrouracil, beta-D-galactosylqueosine, inosine, N6-isopentenyladenine, 1-methylguanine, 1-methylinosine, 2,2-dimethylguanine, 2 methyladenine, 2-methylguanine, 3-methylcytosine, 5-methylcytosine, N6-adenine, 7 methylguanine, 5-methylaminomethyluracil, 5-methoxyaminomethyl-2-thiouracil, beta D-mannosylqueosine, 5'-methoxycarboxymethyluracil, 5-methoxyuracil, 2-methylthio N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5 methyluracil, uracil-5-oxyacetic acid methylester, uracil-5-oxyacetic acid (v), 5-methyl 2-thiouracil, 3-(3- amino-3-N-2-carboxypropyl) uracil, (acp3)w, and 2,6-diaminopurine.

Alternatively, the antisense nucleic acid can be produced biologically using an expression vector into which a nucleic acid has been sub- cloned in an antisense orientation (i.e., RNA transcribed from the inserted nucleic acid will be of an antisense orientation to a target nucleic acid of interest, described further in the following subsection). The antisense nucleic acid molecules of the invention are typically administered to a subject or generated in situ such that they hybridize with or bind to cellular mRNA and/or genomic DNA encoding a polypeptide corresponding to a selected marker gene of the invention to thereby inhibit expression of the marker gene, e.g. by inhibiting transcription and/or translation. The hybridization can be by conventional nucleotide complementarily to forth a stable duplex, or, for example, in the case of an antisense nucleic acid molecule which binds to DNA duplexes, through specific interactions in the major groove of the double helix. Examples of a route of administration of antisense nucleic acid molecules of the invention includes direct injection at a tissue site or infusion of the antisense nucleic acid into a colon- associated body fluid. Alternatively,; antisense nucleic acid molecules can be modified to target selected cells and then administered systemically. For example, for systemic administration, antisense molecules can be modified such that they specifically bind to receptors or antigens expressed on a selected cell surface, e.g. by linking the antisense nucleic acid molecules to peptides or antibodies which bind to cell surface receptors or antigens. The antisense nucleic acid molecules can also be delivered to cells using the vectors described herein.

To achieve sufficient intracellular concentrations of the antisense molecules, vector constructs in which the antisense nucleic acid molecule is placed under the control of a strong pol II or pot III promoter are preferred.

An antisense nucleic acid molecule of the invention can be an a-anomeric nucleic acid molecule. An anomeric nucleic acid molecule forms specific double-stranded hybrids with complementary RNA in which, contrary to the usual a- units, the strands run parallel to each other (Gautier, C., et al., Nucleic Acids Res. 15 (1987) 6625-6641). The antisense nucleic acid molecule can also comprise a 2'-o-methylribonucleotide (Inoue, H., et al., Nucleic Acids Res. 15 (1987) 6131-6148) or a chimeric RNA-DNA analogue (Inoue, H., et al., FEBS Lett. 215 (1987) 327-330).

The invention also encompasses ribozymes. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes as described in Haseloff, J., and Gerlach, W.L., Nature 334 (1988) 585-591) can be used to catalytically cleave mRNA transcripts to thereby inhibit translation of the protein encoded by the mRNA. A ribozyme having specificity for a nucleic acid molecule encoding by a marker gene of the invention can be designed based upon the nucleotide sequence of a cDNA corresponding to the marker gene. For example, a derivative of a Tetrahymena L-19 IVS RNA can be constructed in which the nucleotide sequence of the active site is complementary to the nucleotide sequence to be cleaved (see U.S. Patent No. 4,987,071; and U.S. Patent No. 5,116,742). Alternatively, an mRNA encoding a polypeptide of the invention can be used to select a catalytic RNA having a specific ribonuclease activity from a pool of RNA molecules (see, e.g., Bartel, D.P., and Szostak, J.W., Science 261 (1993) 1411-1418).

The invention also encompasses nucleic acid molecules which form triple helical structures. For example, expression of a polypeptide of the invention can be inhibited by targeting nucleotide sequences complementary to the regulatory region of the gene encoding the polypeptide (e.g. the promoter and/or enhancer) to form triple helical structures that prevent transcription of the gene in target cells. See generally Helene, C., Anticancer Drug Des. 6 (1991) 569-584; Helene, C., et al., Ann. N. Y. Acad. Sci. 660 (1992) 27-36; and Maher, L.J. 3rd., Bioassays 14 (1992) 807-815.

In various embodiments, the nucleic acid molecules of the invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (see Hyrup, B., and Nielsen, P.E., Bioorg. Med. Chem. 4 (1996) 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural bases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al., 1996, supra; Perry-O'Keefe, H., et al., Proc. Natl. Acad. Sci. USA 93 (1996) 14670-14675.

PNAs can be used in therapeutic and diagnostic applications. For example, PNAs can be used as antisense or anti-gene agents for sequence-specific modulation of gene expression by, e.g. inducing transcription or translation arrest or inhibiting replication. PNAs can also be used, e.g., in the analysis of single base pair mutations in 2s a gene by, e.g. PNA directed PCR clamping, as artificial restriction enzymes when used in combination with other enzymes, e.g. S1 nucleases (Hyrup, 1996, supra; or as probes or primers for DNA sequence and hybridization (Hyrup, 1996, supra, Perry O'Keefe et al., 1996, supra).

In another embodiment, PNAs can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which can combine the advantageous properties of PNA and DNA. Such chimeras allow DNA recognition enzymes, e.g. RNASE H and DNA polymerases, to interact with the DNA portion while the PNA portion would provide high binding affinity and specificity.

PNA-DNA chimeras can be linked using linkers of appropriate lengths selected in terms of base stacking, number of bonds between the bases, and orientation (Hyrup, 1996, supra). The synthesis of PNA-DNA chimeras can be performed as described in Hyrup (1996), supra, and Finn, P.J., et al., Nucleic Acids Res. 24 (1996) 3357-3363. For example, a DNA chain can be synthesized on a solid support using standard phosphoramidite coupling chemistry and modified nucleoside analogs. Compounds such as 5'-(4 5 methoxytrityl)amino-5'- deoxy-thymidine phosphoramidite can be used as a link between the PNA and the 5' end of DNA (Mag, M., and Engels, J.W., Nucleic Acids Res. 17 (1989) 5973-5988).

PNA monomers are then coupled in a step-wise manner to produce a chimeric molecule with a 5' PNA segment and a 3' DNA segment (Finn, P.J., et al., Nucleic Acids Res. 24 (1996) 3357-3363). Alternatively, chimeric molecules can be synthesized with a 5' DNA segment and a 3' PNA segment (Peterser et al., Bioorganic Med. Chem. Lett. 5 (1975) 1119-11124).

In other embodiments, the oligonucleotide can include other appended groups such as peptides (e.g. for targeting host cell receptors in viva), or agents facilitating transport across the cell membrane (see, e.g., Letsinger, R.L., et al., Proc. Natl. Acad.Sci. USA 86 (1989) 6553-6556; Lemaitre, M., et al., Proc. Natl. Acad. Sci. USA 84 (1987) 648-652; WO 88/09810) or the blood-brain barrier (see, e.g., WO 89/10134). In addition, oligonucleotides can be modified with hybridization-triggered cleavage agents (see, e.g., van der Krol, A.R., et al., Bio/Techniques 6 (1988) 958-976) or intercalating agents (see, e.g., Zon, G., Pharm. Res. 5 (1988) 539-549). To this end, the oligonucleotide can be conjugated to another molecule, e.g., a peptide, hybridization triggered cross-linking agent, transport agent, hybridization-triggered cleavage agent, etc. The invention also includes molecular beacon nucleic acids having at least one region which is complementary to a nucleic acid of the invention, such that the molecular beacon is useful for quantitating the presence of the nucleic acid of the invention in a sample. A "molecular beacon" nucleic acid is a nucleic acid comprising a pair of complementary regions and having a fluorophore and a fluorescent quencher associated therewith. The fluorophore and quencher are associated with different portions of the nucleic acid in such an orientation that when the complementary regions are annealed with one another, fluorescence of the fluorophore is quenched by the quencher. When the complementary regions of the nucleic acid are not annealed with one another, fluorescence of the fluorophore is quenched to a lesser degree. Molecular beacon nucleic acids are described, for example, in U.S. Patent 5,876,930.

### Isolated Proteins and Antibodies

The following paragraph is generally directed to the proteins used in the present invention and how to obtain them:

One aspect of the invention pertains to isolated proteins encoded by individual marker genes of the invention, and biologically active portions thereof, as well as polypeptide fragments suitable for use as immunogens to raise antibodies directed against a polypeptide encoded by a marker gene of the invention. In one embodiment, the native polypeptide encoded by a marker gene can be isolated from cells or tissue sources by an appropriate purification scheme using standard protein purification techniques. In another embodiment, polypeptides encoded by a marker gene of the invention are produced by recombinant DNA techniques. Alternative to recombinant expression, a polypeptide encoded by a marker gene of the invention can be synthesized chemically using standard peptide synthesis techniques.

An "isolated" or "purified" protein or biologically active portion thereof is substantially free of cellular material or other contaminating proteins from the cell or tissue source from which the,protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%,10%, or 5 % (by dry weight) of heterologous protein (also referred to herein as a "contaminating protein").

When the protein or biologically active portion thereof is recombinantly produced, it is also preferably substantially free of culture medium, i. e., culture medium represents less than about 20 %,10 %, or 5 % of the volume of the protein preparation. When the protein is produced by chemical synthesis, it is preferably substantially free of chemical precursors or other chemicals, i. e., it is separated from chemical precursors or other chemicals which are involved in the synthesis of the protein. Accordingly such preparations of the protein have less than about 30 %,20 %, 10 %, 5 % (by dry weight) of chemical precursors or compounds other than the polypeptide of interest.

Biologically active portions of a polypeptide encoded by a marker gene of the invention include polypeptides comprising amino acid sequences sufficiently identical to or derived from the amino acid sequence of the protein encoded by the marker gene (e.g., the amino acid sequence listed in the GenBank and IMAGE Consortium database records described herein), which include fewer amino acids than the full length protein, and exhibit at least one activity of the corresponding full-length protein. Typically, biologically active portions comprise a domain or motif with at least one activity of the corresponding protein. A biologically active portion of a protein of the invention can be a polypeptide which is, for example, 10, 25, 50, 100 or more amino acids in length.

Moreover, other biologically active portions, in which other regions of the protein are deleted, can be prepared by recombinant techniques and evaluated for one or more of the functional activities of the native form of a polypeptide of the invention. Preferred polypeptides have the amino acid sequence listed in the NCBI Protein Database records described herein. Other useful proteins are substantially identical (e.g. at least about 40%, preferably 50%, 60%, 70%, 80%, 90%, 95%, or 99%) to one of these sequences and retain the functional activity of the protein of the corresponding naturally-occurring protein yet differ in amino acid sequence due to natural allelic variation or mutagenesis.

To determine the percent identity of two amino acid sequences or of two nucleic acids, the sequences are aligned for optimal comparison purposes (e.g. gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i. e., % identity = of identical positions/total of positions (e.g. overlapping positions) x100).

In one embodiment the two sequences are the same length.

The determination of percent identity between two sequences can be accomplished using a mathematical algorithm. A preferred, non-limiting example of a mathematical algorithm utilized for the comparison of two sequences is the algorithm of Karlin, S., and Altschul, S.F., Proc. Natl. Acad. Sci. USA 87 (1990) 2264-2268, modified as in Karlin, S., and Altschul, S.F., Proc. Natl. Acad. Sci. USA 90 (1993) 5873-5877. Such an algorithm is incorporated into the NBLAST and XBLAST programs of Altschul, S.F., et al., J. Mol. Biol. 215 (1990) 403-410. BLAST nucleotide searches can be performed with the NBLAST program, score = 100, wordlength = 12 to obtain nucleotide sequences homologous to a nucleic acid molecules of the invention. BLAST protein searches can be performed with the XBLAST program, score = 50, wordlength = 3 to obtain amino acid sequences homologous to a protein molecules of the invention. To obtain gapped alignments for comparison purposes, Gapped BLAST can be utilized as described in Altschul, S.F., et al., Nucleic Acids Res. 25 (1997) 3389-3402. Alternatively, PSI-Blast can be used to perform an iterated search which detects distant relationships between molecules. When utilizing BLAST, Gapped BLAST, and PSI-Blast programs, the default parameters of the respective programs (e.g., XBLAST and NBLAST) can be used. See http://www.ncbi.nlm.nih.gov. Another preferred, non-limiting example of a mathematical algorithm utilized for the comparison of sequences is the algorithm of Myers and Miller, (1988) CABIOS or 11 - 17. Such an algorithm is incorporated into the ALIGN program (version 2.0) which is part of the GCG sequence alignment software package. When utilizing the ALIGN program for comparing amino acid sequences, a PAM 120 weight residue table, a gap length penalty of 12, and a gap penalty of 4 can be used. Yet another useful algorithm for identifying regions of local sequence similarity and alignment is the FASTA algorithm as described in Pearson and Lipman (1988) Proc. Natl. A cad. Sci. USA 85:2444-2448. When using the PASTA algorithm for comparing nucleotide or amino acid sequences, a PAM120 weight residue table can, for example, be used with a k-tuple value of 2.

The percent identity between two sequences can be determined using techniques similar to those described above, with or without allowing gaps. In calculating percent identity, only exact matches are counted.

The invention also provides chimeric or fusion proteins corresponding to a marker gene of the invention. As used herein, a "chimeric protein" or "fusion protein" comprises all or part (preferably a biologically active part) of a polypeptide encoded by a marker gene of the invention operably linked to a heterologous polypeptide (i. e., a polypeptide other than the polypeptide encoded by the marker gene). Within the fusion protein, the term "operably linked" is intended to indicate that the polypeptide of the invention and the heterologous polypeptide are fused in-frame to each other. The heterologous polypeptide can be fused to the amino-terminus or the carboxyl-terminus of the polypeptide of the invention.

One useful fusion protein is a GST fusion protein in which a polypeptide encoded by a marker gene of the invention is fused to the carboxyl terminus of GST 2s sequences. Such fusion proteins can facilitate the purification of a recombinant polypeptide of the invention.

In another embodiment, the fusion protein contains a heterologous signal sequence at its amino terminus. For example, the native signal sequence of a polypeptide encoded by a marker gene of the invention can be removed and replaced with a signal sequence from another protein. For example, the gp67 secretory sequence of the baculovirus envelope protein can be used as a heterologous signal sequence (Ausubel et al., ed., Current Protocols in Molecular Biology, John Wiley & Sons, New York, 1987-1999). Other examples of eukaryotic heterologous signal sequences include the secretory sequences of melittin and human placental alkaline phosphatase (Stratagene; La Jolla, California). In yet another example, useful prokaryotic heterologous signal sequences include the phoA secretory signal (Sambrook et al., supra) and the protein A secretory signal (Pharmacia Biotech; Piscataway, New Jersey).

In yet another embodiment, the fusion protein is an immunoglobulin fusion protein in which all or part of a polypeptide encoded by a marker gene of the invention is fused to sequences derived from a member of the immunoglobulin protein family.

The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in viva. The immunoglobulin fusion protein can be used to affect the bioavailability of a cognate ligand of a polypeptide of the invention. Inhibition of ligand/receptor interaction can be useful therapeutically, both for treating proliferative and differentiative disorders and for modulating (e.g promoting or inhibiting) cell survival. Moreover, the immunoglobulin fusion proteins of the invention can be used as immunogens to produce antibodies directed against a polypeptide of the invention in a subject, to purify ligands and in screening assays to identify molecules which inhibit the interaction of receptors with ligands.

Chimeric and fusion proteins of the invention can be produced by standard recombinant DNA techniques. In another embodiment, the fusion gene can be synthesized by conventional techniques including automated DNA synthesizers.

Alternatively, PCR amplification of gene fragments can be carried out using anchor primers which give rise to complementary overhangs between two consecutive gene fragments which can subsequently be annealed and re-amplified to generate a chimeric gene sequence (see, e.g. Ausubel et al., supra). Moreover, many expression vectors are commercially available that already encode a fusion moiety (e.g. a GST polypeptide).

A nucleic acid encoding a polypeptide of the invention can be cloned into such an expression vector such that the fusion moiety is linked in-frame to the polypeptide of the invention.

A signal sequence can be used to facilitate secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the invention pertains to the described polypeptides having a signal sequence, as well as to polypeptides from which the signal sequence has been proteolytically cleaved (i. e., the cleavage products). In one embodiment, a nucleic acid sequence encoding a signal sequence can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods.

Alternatively, the signal sequence can be linked to the protein of interest using a sequence which facilitates purification, such as with a GST domain.

The present invention also pertains to variants of the polypeptides encoded by individual marker genes of the invention. Such variants have an altered amino acid sequence which can function as either agonists (mimetics) or as antagonists. Variants can be generated by mutagenesis, e. g. discrete point mutation or truncation. An agonist can retain substantially the same, or a subset, of the biological activities of the naturally occurring Loran of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the protein.

Variants of a protein of the invention which function as either agonists (mimetics) or as antagonists can be identified by screening combinatorial libraries of mutants, e.g. truncation mutants, of the protein of the invention for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (e.g., for phage display). There are a variety of methods which can be used to produce libraries of potential variants of the polypeptides of the invention from a degenerate oligonucleotide sequence. Methods for synthesizing degenerate oligonucleotides are known in the art (see, e.g., Narang, Tetrahedron 39 (1983) 3; Itakura, K., et al., Annu. Rev. Biochem. 53 (1984) 323-356; Itakura, K., et al., Science 198 (1977) 1056-1063; Ike, Y., et al., Nucleic Acid Res. 11 (1983) 477-488).

In addition, libraries of fragments of the coding sequence of a polypeptide encoded by a marker gene of the invention can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S 1 nuclease, and ligating the resulting fragment library into an expression vector. By this method, an expression library can be derived which encodes amino terminal and internal fragments of various sizes of the protein of interest.

Several techniques are known in the art for screening gene products of combinatorial libraries made by point mutations or truncation, and for screening cDNA libraries for gene products having a selected property. The most widely used techniques, which are amenable to high through- put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of a protein of the invention (Arkin, A.P., and Youvan, D.C., Proc. Natl. Acad. Sci. USA 89 (1992) 7811-7815; Delagrave, S., et al., Prot. Eng. 6 (1993) 327-331).

An isolated polypeptide encoded by a marker gene of the invention, or a fragment thereof, can be used as an immunogen to generate antibodies using standard techniques for polyclonal and monoclonal antibody preparation. The full-length polypeptide or protein can be used or, alternatively, the invention provides antigenic peptide fragments for use as immunogens. The antigenic peptide of a protein of the invention comprises at least 8 (preferably 10, 15, 20, or 30 or more) amino acid residues of the amino acid sequence of one of the polypeptides of the invention, and encompasses an epitope of the protein such that an antibody raised against the peptide forms a specific immune complex with a protein encoded by a marker gene of the invention. Preferred epitopes encompassed by the antigenic peptide are regions that are located on the surface of the protein, e.g. hydrophilic regions. Hydrophobicity sequence analysis, hydrophilicity sequence analysis, or similar analyses can be used to identify hydrophilic j regions. An immunogen typically is used to prepare antibodies by immunizing a suitable (i.e. immunocompetent) subject such as a rabbit, goat, mouse, or other mammal or vertebrate. An appropriate immunogenic preparation can contain, for example, recombinantly-expressed or chemically-synthesized polypeptide. The preparation can further include an adjuvant, such as Freund's complete or incomplete adjuvant, or a similar immunostimulatory agent.

Accordingly, another aspect of the invention pertains to antibodies directed against a polypeptide of the invention. The terms "antibody" and "antibody substance"; as used interchangeably herein refer to immunoglobulin molecules and immunologically active portions of immunoglobulin molecules, i.e., molecules that contain an antigen binding site which specifically binds an antigen, such as a polypeptide of the invention, e.g., an epitope of a polypeptide of the invention. A molecule which specifically binds to a given polypeptide of the invention is a molecule which binds the polypeptide, but does not substantially bind other molecules in a sample, e.g. a biological sample, which naturally contains the polypeptide. Examples of immunologically active portions of immunoglobulin molecules include F(ab) and F(ab')2 fragments which can be generated by treating the antibody with an enzyme such as pepsin. The invention provides polyclonal and monoclonal antibodies. The term "monoclonal antibody" or "monoclonal antibody composition", as used herein, refers to a population of antibody molecules that contain only one species of an antigen binding site capable of immunoreacting with a particular epitope.

Polyclonal antibodies can be prepared as described above by immunizing a suitable subject with a polypeptide of the invention as an immunogen. Preferred polyclonal antibody compositions are ones that have been selected for antibodies directed against a polypeptide or polypeptides of the invention. Particularly preferred polyclonal antibody preparations are ones that contain only antibodies directed against a polypeptide or polypeptides of the invention. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be harvested or isolated from the subject (e.g., from the blood or serum of the subject) and further purified by well-known techniques, such as protein A chromatography to obtain the IgG fraction. Alternatively, antibodies specific for a protein or polypeptide of the invention can be selected or (e.g., partially purified) or purified by, e.g. affinity chromatography.

For example, a recombinantly expressed and purified (or partially purified) protein of the invention is produced as described herein, and covalently or non-covalently coupled to a solid support such as, for example, a chromatography colunan. The column can then be used to affinity purify antibodies specific for the proteins of the invention from a sample containing antibodies directed against a large number of different epitopes, thereby generating a substantially purified antibody composition, i. e., one that is substantially free of contaminating antibodies. By a substantially purified antibody composition is meant, in this context, that the antibody sample contains at most only 30% (by dry weight) of contaminating antibodies directed against epitopes other than those of the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, e.g., when the specific antibody titers are highest, antibody-producing cells can be obtained from the subject and used to prepare monoclonal antibodies by standard techniques, such as the hybridoma technique originally described by Kohler, G., and Milstein, C., Nature 256 (1975) 495-497, the human B cell hybridoma technique (see Kozbor et al., Immunol. Today 4 (1983) 72), the EBV hybridoma technique (see Cole et al., In: Monoclonal Antibodies and Cancer Therapy, Alan R. Liss, Inc., 1985, pp. 77-96) or trioma techniques. The technology for producing hybridomas is well known (see generally Coligan et al., ed., Current Protocols in Immunology, John Wiley & Sons, New York, 1994). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g. an antibody phage display library) with the polypeptide of interest. Kits for generating and screening phage display libraries are commercially available (e.g. the Pharmacia Recombinant Phage Antibody System, Catalog No. 27-9400-01; end the Stratagene Su' 4P Phage Display Kit, Catalog No. 240612). Additionally, examples of methods and reagents particularly amenable for use in generating and screening antibody display library can be found in, for example, U.S. Patent No. 5,223,409; WO 92/18619; WO 91/17271; WO 92/20791; WO 92/15679; WO 93/01288; WO 92/01047; WO 92/09690; WO 90/02809; Fuchs, P., et al. Bio/Technology 9 ((1991)) 1369-1372; Hay, B.N., et al., Hum. Antibod. Hybridomas 3 (1992) 81-85; Huse, W.D., et al., Science 246 (1989) 1275-1281; Griffiths, A.D., et al., EMBO 12 (1993) 725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, e.g., U.S. Patent No. 4,816,567; and U.S. Patent No. 4,816,397, which are incorporated herein by reference in their entirety.) Humanized antibodies are antibody molecules from non-human species having one or more complementarily determining 1o regions (CDRs) from the non-human species and a framework region from a human immunoglobulin molecule. (See, e.g., U.S. Patent No. 5,585,089, which is incorporated herein by reference in its entirety.) Such chimeric and humanized monoclonal antibodies can be produced by recombinant DNA techniques known in the art, for example using methods described in WO 87/02671; EP-A 0 184 187; EP-A 0 171 496; EP-A 0 173 494; WO 86/01533; U.S. Patent No. 4,816,567; EP-A 0 125 023; Better, M., et al., Science 240 (1988) 1041-1043; Liu, A.Y., et al., Proc. Natl. Acad. Sci. USA 84 (1987) 3439-3443; Liu, A.Y., et al., J. Immunol. 139 (1987) 3521-3526; Sun, L.K., et al., Proc. Natl. Acad. Sci. USA 84 ((1987)) 214-218; Nishimura, Y., et al., Cancer Res. 47 (1987) 999-1005; Wood, C.R., et al., Nature 314 (1985) 446-449; and Shaw, D.R., et al., Natl. Cancer Inst. 80 (1988) 1553-1559; Morrison, S.L., Science 229 (1985) 1202-1207; Oi et al., Bio/Techniques 4 (1986) 214; U.S. Patent 5,225,539; Jones, P.T., et al., Nature 321 (1986) 522-525; Verhoeyen, M., et al., Science 239 (1988) 1534-1536; and Beidler, C.B., et al., J. Immunol. 141 (1988) 4053-4060.

Antibodies of the invention may be used as therapeutic agents in treating cancers. In a preferred embodiment, completely human antibodies of the invention are used for therapeutic treatment of human cancer patients, particularly those having breast cancer. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g. all or a portion of a polypeptide encoded by a marker gene of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation.

Thus, using such a technique, it is possible to produce therapeutically useful IgG, IgA and IgE antibodies. For an overview of this technology for producing human antibodies, see Lonberg, N., and Huszar, D., Int. Rev. Immunol. 13 (1995) 65-93). For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope (Jespers, L.S., et al., Bio/Technology 12 (1994) 899-903).

An antibody directed against a polypeptide encoded by a marker gene of the invention (e.g. a monoclonal antibody) can be used to isolate the polypeptide by standard techniques, such as affinity chromatography or immunoprecipitation.

Moreover, such an antibody can be used to detect the polypeptide (e.g., in a cellular lysate or cell supernatant) in order to evaluate the level and pattern of expression of the marker gene. The antibodies can also be used diagnostically to monitor protein levels in tissues or body fluids (e.g. in an ovary-associated body fluid) as part of a clinical testing procedure, e.g. to, for example, determine the efficacy of a given treatment regimen.

Detection can be facilitated by coupling the antibody to a detectable substance.

Examples of detectable substances include various enzymes, prosthetic groups, fluorescent materials, luminescent materials, bioluminescent materials, and radioactive materials. Examples of suitable enzymes include horseradish peroxidase, alkaline phosphatase, 6-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include I, P, S or H. Further, an antibody (or fragment thereof) can be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B. gramicidin D, ethidium bromide, emetine, mitomycin, etoposide, tenoposide, vincristine, vinblastine, colchicin, doxorubicin, daunorubicin, dThydroxy anthracin dione, mitoxantrone, mithramycin, actinomycin D, 1-dehydrotestosterone, - 46 glucocorticoids, procaine, tetracaine, lidocaine, propranolol, and puromycin and analogs or homologs thereof. Therapeutic agents include, but are not limited to, antimetabolites (e.g., methotrexate, 6- mercaptopurine, 6-thioguanine, cytarabine, 5-fluorouracil decarbazine), alkylating agents (e.g. mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g. daunorubicin (formerly daunomycin) and doxorubicin), antibiotics (e.g. dactinomycin (formerly actinomycin), bleomycin, mithramycin, and anthramycin (AMC)), and anti-mitotic agents (e.g. vincristine and o vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", In: Monoclonal Antibodies And Cancer Therapy, Reisfeld et al. (eds.), Alan R. Liss, Inc., 1985, pp. 243-256; Hellstrom et al., "Antibodies For Drug Delivery", In: Controlled Drug Delivery, 2nd ed., Robinson et al. (eds.), Marcel Dekker, Inc., 1987, pp. 623-653; Thorpe, "Antibody Carriers Of Cytotoxic Agents In Cancer Therapy: A Review", In: Monoclonal Antibodies '84: Biological And Clinical Applications, Pinchera et al. (eds.), 1985, pp. 475-506; Baldwin et al. (eds.), "Analysis, Results, And Future Prospective Of The Therapeutic Use Of Radiolabeled Antibody In Cancer Therapy", In: Monoclonal Antibodies For Cancer Detection And Therapy, Academic Press, 1985, pp. 303-316; and Thorpe, P.E., and Ross, W.C., Immunol. Rev. 62 (1982) 119-158.

Alternatively, an antibody can be conjugated to a second antibody to form an antibody heteroconjugate as described by Segal in U.S. Patent No. 4,676,980.

Accordingly, in one aspect, the invention provides substantially purif ed antibodies or fragments thereof, and non-human antibodies or fragments thereof, which antibodies or fragments specifically bind to a polypeptide comprising an amino acid sequence selected from the group consisting of the amino acid sequences of the present invention, an amino acid sequence encoded by the cDNA of the present invention, a fragment of at least 15 amino acid residues of an amino acid sequence of the present invention, an amino acid sequence which is at least 95% identical to the amino acid sequence of the present invention (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules of the present invention, or a complement thereof, under conditions of hybridization of 6X SSC at 45°C and washing in 0.2 X SSC, 0.1% SDS at 65°C. In various embodiments, the substantially purified antibodies of the invention, or fragments thereof, can be human, non-human, chimeric and/or; humanized antibodies.

In another aspect, the invention provides non-human antibodies or fragments thereof, which antibodies or fragments specifically bind to a polypeptide comprising an amino acid sequence selected from the group consisting of: the amino acid sequence of the present invention, an amino acid sequence encoded by the cDNA of the present invention, a fragment of at least 15 amino acid residues of the amino acid sequence of; the present invention, an amino acid sequence which is at least 95% identical to the amino acid sequence of the present invention (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules of the present invention, or a complement thereof, under conditions of hybridization of 6X SSC at 45°C and washing in 0.2 X SSC, 0.1% SDS at 65°C. Such non- human antibodies can be goat, mouse, sheep, horse, chicken, rabbit, or rat antibodies. Alternatively, the non-human antibodies of the invention can be chimeric and/or humanized antibodies. In addition, the non human antibodies of the invention can be polyclonal antibodies or monoclonal antibodies.

In still a further aspect, the invention provides monoclonal antibodies or fragments thereof, which antibodies or fragments specifically bind to a polypeptide comprising an amino acid sequence selected from the group consisting of the amino acid sequences of the present invention, an amino acid sequence encoded by the cDNA of the present invention, a fragment of at least 15 amino acid residues of an amino acid sequence of the present invention, an amino acid sequence which is at least 95% identical to an amino acid sequence of the present invention (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules of the present invention or a complement thereof, under conditions of hybridization of 6X SSC at 45°C and washing in 0.2 X SSC, 0.1% SDS at 65°C. The monoclonal antibodies can be human, humanized, chimeric and/or non-human antibodies.

The substantially purif ed antibodies or fragments thereof may specie catty bind to a signal peptide, a secreted sequence, an extracellular domain, a kansmembrane or a cytoplasmic domain or cytoplasmic membrane of a polypeptide of the invention. In a particularly preferred embodiment, the substantially purified antibodies or fragments thereof, the non-human antibodies or fragments thereof, and/or the monoclonal; antibodies or fragments thereof, of the invention specifically bind to a secreted sequence or an extracellular domain of the amino acid sequences of the present invention.

Any of the antibodies of the invention can be conjugated to a therapeutic moiety; or to a detectable substance. Non-limiting examples of detectable substances that can be conjugated to the antibodies of the invention are an enzyme, a prosthetic group, a fluorescent material, a luminescent material, a bioluminescent material, and a radioactive material.

The invention also provides a kit containing an antibody of the invention conjugated to a detectable substance, and instructions for use. Still another aspect of the invention is a pharmaceutical composition comprising an antibody of the invention and a pharmaceutically acceptable carrier. In preferred embodiments, the pharmaceutical composition contains an antibody of the invention, a therapeutic moiety, and a pharmaceutically acceptable carrier.

Still another aspect of the invention is a method of making an antibody that specifically recognizes a polypeptide of the present invention, the method comprising immunizing a mammal with a polypeptide. The polypeptide used as an immunogen comprises an amino acid sequence selected from the group consisting of the amino acid sequence of the present invention, an amino acid sequence encoded by the cDNA of the nucleic acid molecules of the present invention, a fragment of at least 15 amino acid residues of the amino acid sequence of the present invention, an amino acid sequence which is at least 95% identical to the amino acid sequence of the present invention (wherein the percent identity is determined using the ALIGN program of the GCG software package with a PAM120 weight residue table, a gap length penalty of 12, and a gap penalty of 4) and an amino acid sequence which is encoded by a nucleic acid molecule which hybridizes to a nucleic acid molecule consisting of the nucleic acid molecules of the present invention, or a complement thereof, under conditions of hybridization of 6X SSC at 45°C and washing in 0.2 X SSC, 0.1% SDS at 65°C.

After immunization, a sample is collected from the mammal that contains an antibody that specifically recognizes the polypeptide. Preferably, the polypeptide is recombinantly produced using a non-human host cell. Optionally, the antibodies can be further purified from the sample using techniques well known to those of skill in the art.

The method can further comprise producing a monoclonal antibody- producing cell from the cells of the mammal. Optionally, antibodies are collected from the antibody-producing cell.

### Recombinant Expression Vectors and Host Cells

The following paragraph is generally directed to recombinant expression vectors and how to obtain them for producing the proteins according to the invention:

Another aspect of the invention pertains to vectors, preferably expression vectors, containing a nucleic acid encoding a polypeptide encoded by a marker gene of the invention (or a portion of such a polypeptide). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g. bacterial vectors having a bacterial origin of replication and episomal mammalian vectors). Other vectors (e.g. non-episomal mammalian vectors) are integrated into the genome of a host cell upon introduction into the host cell, and thereby are replicated along with the host genome. Moreover, certain vectors, namely expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e. g. replication defective retroviruses, adenoviruses and adeno-associated viruses), which serve equivalent functions.; The recombinant expression vectors of the invention comprise a nucleic acid of the invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g. in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel, Methods in Enzymology: Gene Expression Technology, Vol. 185, Academic Press, San Diego, CA, 1991. Regulatory sequences include those which direct constitutive expression of a nucleotide sequence in many types of host cell and those which direct expression of the nucleotide sequence only in certain host cells (e.g. tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as; described herein.

The recombinant expression vectors of the invention can be designed for is expression of a polypeptide encoded by a marker gene of the invention in prokaryotic (e.g. E. coli) or eukaryotic cells (end insect cells {using baculovirus expression vectors}, yeast cells or mammalian cells). Suitable host cells are discussed further in Goeddel, supra. Alternatively, the recombinant expression vector can be transcribed; and translated in vitro, for example using T7 promoter regulatory sequences and T7 polymerase.

Expression of proteins in prokaryotes is most often carried out in E. coli with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D.B., and Johnson, K.S., Gene 67 (1988) 31-40), pMAL (New England Biolabs, Beverly, MA) and pRIT5 (Phannacia, Piscataway, NJ) which fuse glutathione S-transferase (GST),; maltose E binding protein, or protein A, respectively, to the target recombinant protein.

Examples of suitable inducible non-fusion E. cold expression vectors include pTrc (Amann, E., et al., Gene 69 (1988) 301-315) and pET 1 Id (Studier et al., In: Gene Expression Technology: Methods in Enzymology, Vol. 85, Academic Press, San Diego, CA, 1991, pp. 60-89). Target gene expression from the pTrc vector relies on host RNA polymerase transcription from a hybrid trp-lac fusion promoter. Target gene expression from the pET 1 Id vector relies on transcription from a T7 gulO-lac fusion promoter mediated by a co-expressed viral RNA polymerase (T7 gal). This viral polymerase is supplied by host strains BL21(DE3) or HMS 174(DE3) from a resident prophage harboring a T7 gal gene under the transcriptional control of the lacUV promoter.

One strategy to maximize recombinant protein expression in E. coli is to express the protein in a host bacteria with an impaired capacity to proteolytically cleave the recombinant protein (Gottesman, In: Gene Expression Technology: Methods in Enzymology, Vol. 185, Academic Pres, San Diego, CA, 1990, pp. 119-128). Another strategy is to alter the nucleic acid sequence of the nucleic acid to be inserted into an expression vector so that the individual codons for each amino acid are those preferentially utilized in E. coli(Wada, K., et al., Nucleic Acids Res. 20 (1992) 2111-2118). Such alteration of nucleic acid sequences of the invention can be carried out by standard DNA synthesis techniques. In another embodiment, the expression vector is a yeast expression vector.

Examples of vectors for expression in yeast S. cerevisiae include pYepSecl (Baldari, C., et al., EMBO J. 6 (1987) 229-234), pMFa (Kurjan, J., and Herskowitz, I., Cell 30 (1982) 933-943), pJRY88 (Schultz, L.D., et al., Gene 54 (1987) 113-123), pYES2 (Invitrogen Corporation, San Diego, CA), and pPicZ (Invitrogen Corp, San Diego, CA).

Alternatively, the expression vector is a baculovirus expression vector.

Baculovirus vectors available for expression of proteins in cultured insect cells (e.g. Sf 9 cells) include the pAc series (Smith, G.E., et al., Mol. Cell Biol. 3 (1983) 2156-2165) and the pVL series (Luckow, V.A., and Summers, M.D., Virology 170 (1989) 31-39).

In yet another embodiment, a nucleic acid of the invention is expressed in mammalian cells using a mammalian expression vector. Examples of mammalian expression vectors include pCDM8 (Seed, B., Nature 329 (1987) 840-842) and pMT2NOPC (Kaufman, R.J., et al., EMBO J. 6 (1987) 187-193). When used in mammalian cells, the expression vector's control functions are often provided by viral regulatory elements.

For example, commonly used promoters are derived from polyoma, Adenovirus 2, cytomegalovirus and Simian Virus 40. For other suitable expression systems for both; prokaryotic and eukaryotic cells see chapters 16 and 17 of Sambrook et al., supra.

In another embodiment, the recombinant mammalian expression vector is capable of directing expression of the nucleic acid preferentially in a particular cell type (e.g. tissue-specific regulatory elements are used to express the nucleic acid). Tissue specific regulatory elements are known in the art. Non-limiting examples of suitable tissue-specific promoters include the albumin promoter (liver-specific; Pinkert, C.A., et al., Genes Dev. 1 (1987) 268-277), lymphoid-specific promoters (Calame, K., and Eaton, S., Adv. Immunol. 43 (1988) 235-275), in particular promoters of T cell receptors (Winoto, A., and Baltimore, D., EMBO J. 8 (1989) 729-733) and immunoglobulins (Banerji, J., et al., Cell 33 (1983) 729-740; Queen, C., and Baltimore, D., Cell 33 (1983) 741-748), neuron-specific promoters (e.g. the neurofilament promoter; Byrne, G.W., and Ruddle, F.H., Proc. Natl. Acad. Sci. USA 86 (1989) 5473-5477), pancreas-specific promoters (Edlund, T., et al., Science 230 (1985) 912-916), and mammary gland-specific promoters (e.g. milk whey promoter, U.S. Patent No. 4,873,316 and EP-A 0 264 166). Developmentally regulated promoters are also encompassed, for example the murine box promoters (Kessel, M., and Gruss, P., Science 249 (1990) 374-379) and the a-fetoprotein promoter (Camper, S.A., and Tilghman, S.M., Genes Dev. 3 (1989) 537-546).

The invention further provides a recombinant expression vector comprising a DNA molecule of the invention cloned into the expression vector in an antisense orientation. That is, the DNA molecule is operably linked to a regulatory sequence in a manner which allows for expression (by transcription of the DNA molecule) of an RNA molecule which is antisense to the mRNA encoding a polypeptide of the invention.

Regulatory sequences operably linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue-specific or cell type specific expression of antisense RNA. The antisense expression vector can be in the form of a recombinant plasmid, phagemid, or attenuated virus in which antisense nucleic acids are produced under the control of a high efficiency regulatory region, the activity of which can be determined by the cell type into which the vector is introduced. For a discussion of the regulation of gene expression, using antisense genes see Weintraub et al., 1986, Trends in Genetics, Vol. 1(1).

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic (e.g. E. colt) or eukaryotic cell (e.g. insect cells, yeast or mammalian cells).

Vector DNA can be introduced into prokaryotic or eukaryotic cells via conventional transformation or transfection techniques. As used herein, the terms "transformation" and "transfection" are intended to refer to a variety of art-recognized; techniques for introducing foreign nucleic acid into a host cell, including calcium phosphate or calcium chloride co- precipitation, DEAE-dextran-mediated transfection, lipofection, or electroporation. Suitable methods for transforming or transfecting host cells can be found in Sambrook, et al. (supra), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a "selectable marker" (SM) gene (e.g., for resistance to antibiotics) is generally introduced into the host cells along with the gene of interest.

Preferred SM genes include those which confer resistance to drugs, such as G418, hygromycin and methotrexate. Cells stably transfected with the introduced nucleic acid can be identified by drug selection (e.g. cells that have incorporated the SM gene will survive, while the other cells die).

A host cell of the invention, such as a prokaryotic or eukaryotic host cell in culture, can be used to produce a polypeptide encoded by a marker gene of the invention. Accordingly, the invention further provides methods for producing a polypeptide encoded by a marker gene of the invention using the host cells of the invention. In one embodiment, the method comprises culturing the host cell of invention (into which a recombinant expression vector encoding a polypeptide of the invention has been introduced) in a suitable medium such that the polypeptide encoded by the marker gene is produced. In another embodiment, the method further comprises isolating the polypeptide from the medium or the host cell. The host cells of the invention can also be used to produce nonhuman transgenic animals. For example, in one embodiment, a host cell of the invention is a fertilized oocyte or an embryonic stem cell into which a sequences encoding a polypeptide of a marker gene of the invention have been introduced. Such host cells can then be used to create non-human transgenic animals in which exogenous sequences encoding a marker gene of the invention have been introduced into their genome or homologous recombinant animals in which endogenous gene(s) encoding a polypeptide corresponding to a marker gene of the invention have been altered. Such animals are useful for studying the function and/or activity of the polypeptide corresponding to the marlcer gene and for identifying and/or evaluating modulators of polypeptide activity.

As used herein, a "transgenic animal" is a non-human animal, preferably a mammal, more preferably a rodent such as a rat or mouse, in which one or more of the cells of the s animal includes a transgene. Other examples of transgenic animals include non-human primates, sheep, dogs, cows, goats, chickens, amphibians, etc. A transgene is exogenous DNA which is integrated into the genome of a cell from which a transgenic animal; develops and which remains in the genome of the mature animal, thereby directing the expression of an encoded gene product in one or more cell types or tissues of the transgenic animal. As used herein, an "homologous recombinant animal" is a non human animal, preferably a mammal, more preferably a mouse, in which an endogenous gene has been altered by homologous recombination between the endogenous gene and an exogenous DNA molecule introduced into a cell of the animal, e.g. an embryonic cell of the animal, prior to development of the animal.

A transgenic animal of the invention can be created by introducing a nucleic acid encoding a polypeptide encoded by a marker gene of the invention into the male pronuclei of a fertilized oocyte, e.g. by microinjection, retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to the transgene to direct expression of the polypeptide of the invention to particular cells. Methods for generating transgenic animals via embryo manipulation and microinjection, particularly animals such as mice, have become conventional in the art and are described, for example, in U.S. Patent Nos. 4,736,866 and 4,870,009, U.S. Patent No. 4,873,191 and in Hogan, Manipulating the Mouse Embryo, Cold Spring Harbor Laboratory Press, Cold Spring Harbor, N.Y., 1986. Similar methods are used for production of other transgenic animals. A transgenic founder animal can be identified based upon the presence of the transgene in its genome and/or expression of mRNA encoding the transgene in tissues or cells of the animals. A transgenic founder animal can then be used to breed additional animals carrying the transgene. Moreover, transgenic animals carrying the transgene can further be bred to other, transgenic animals carrying other transgenes.

To create a homologous recombinant animal, a vector is prepared which contains at least a portion of a marker gene of the invention into which a deletion, addition or substitution has been introduced to thereby alter, e.g. functionally disrupt, the gene. In a preferred embodiment, the vector is designed such that, upon homologous recombination, the endogenous gene is functionally disrupted (i. e., no longer encodes a functional protein; also referred to as a "knock out" vector). Alternatively, the vector can be designed such that, upon homologous recombination, the endogenous gene is mutated or otherwise altered but still encodes functional protein (e.g., the upstream regulatory region can be altered to thereby alter the expression of the endogenous protein). In the homologous recombination vector, the altered portion of the gene is flanked at its 5' and 3' ends by additional nucleic acid of the gene to allow for homologous recombination to occur between the exogenous gene carried by the vector and an endogenous gene in an embryonic stem cell. The additional flanking nucleic acid sequences are of sufficient length for successful homologous recombination with the endogenous gene. Typically, several kilobases of flanking DNA (both at the 5' and 3' ends) are included in the vector (see, e.g., Thomas, K.R., and Capecchi, M.R., Cell 51 (1987) 503-512 for a description of homologous recombination vectors). The vector is introduced into an embryonic stem cell line (e.g. by electroporation) and cells in which the introduced gene has homologously recombined with the endogenous gene are selected (see, e.g., Li, E., et al., Cell 69 (1992) 915-926). The selected cells are then injected into a blastocyst of an animal (e.g. a mouse) to form aggregation chimeras (see, e.g., Bradley, Teratocarcinomas and Embryonic Stem Cells, A Practical Approach, Robertson, ed., IRL, Oxford, 1987, pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, A., Curr. Opin. Biotechnol. 2 (1991) 823-829 and in WO 90/11354; WO 91/01140; WO 92/0968; and WO 93/04169.

In another embodiment, transgenic non-human animals can be produced which contain selected systems which allow for regulated expression of the transgene. One example of such a system is the cre/loxP recombinase system of bacteriophage Pi. For a description of the cre/loxP recombinase system, see, e.g., Lakso, M., et al. Proc. Natl. Acad. Sci. USA 89 (1992) 6232-6236. Another example of a recombinase system is the FLP recombinase system of Saccharomyces cerevisiae (O'Gorman, S., et al., Science 251 (1991) 1351-1355). If a cre/loxP recombinase system is used to regulate expression of the transgene, animals containing transgenes encoding both the Cre recombinase and a selected protein are required. Such animals can be provided through the construction of "double" transgenic animals, e.g. by mating two transgenic animals, one containing a transgene encoding a selected protein and the other containing a transgene encoding a recombinase.

Clones of the non-human transgenic animals described herein can also be produced according to the methods described in Wilmut, I., et al., Nature 385 (1997) 810-813 and WO 97/07668 and WO 97/07669.

### Cells and cell lines

The cells in a sample wherefrom the normal level of expression of a marker gene of the invention is determined are either cells from a control subject not afflicted with mammary cancer or cells from a human mammary epithelial cell line. The cells from a control subject should be obtained in the same manner as from the patient afflicted with breast cancer.

The cells from a human mammary epithelial cell line are preferably those that can be commercially obtained from Cambrex Bio Science Walkersville, Inc., Walkersville, Maryland, USA. Methods for obtaining these or other suitable cell human mammary cell lines are described in US 4,423,145. Other suitable cell lines are described in US 4,808,532.

### Description of the Figures

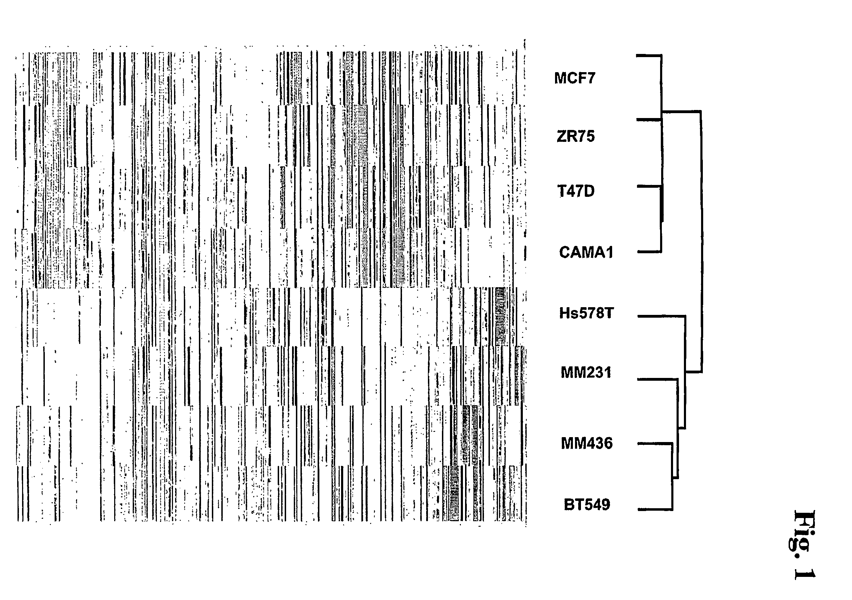

- Fig. 1: Clustering of transcriptional profiles of four invasive versus four non-invasive human mammary carcinoma cell lines.
Total RNA was isolated from the cell lines shown above and the transcriptional profile was determined by means of the Affymetrix GeneChip^{®} Technology. Scoring criteria were as described in the Materials and Methods section. The expression profile of HMEC (Human Mammary Epithelial Cells) was used for generation of a baseline. Clustering was performed with the Eisen software. Red colour shows increased expression, green colour indicates decreased expression of individual genes in the mammary carcinoma cell lines under investigation compared to HMEC cells.

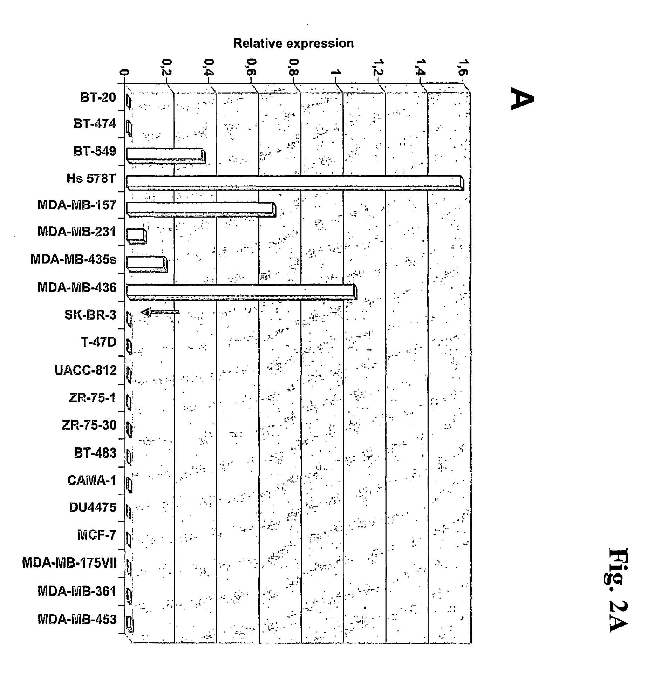

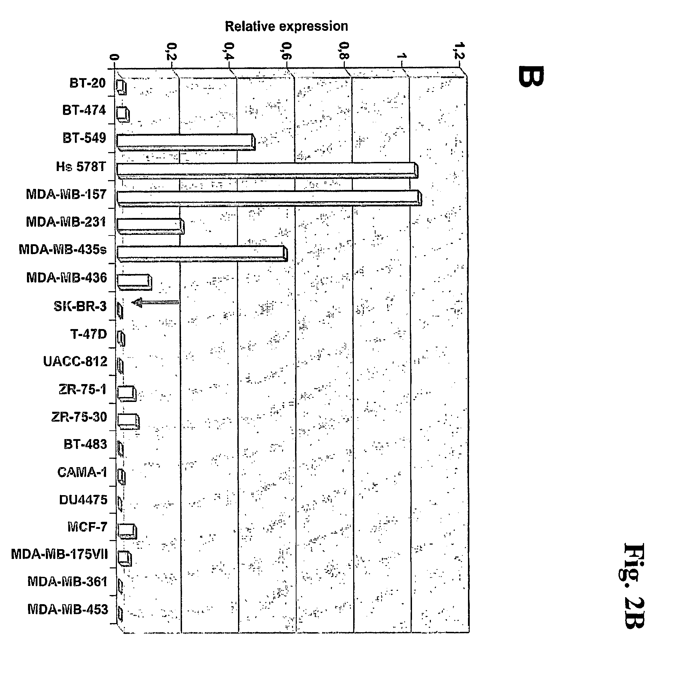

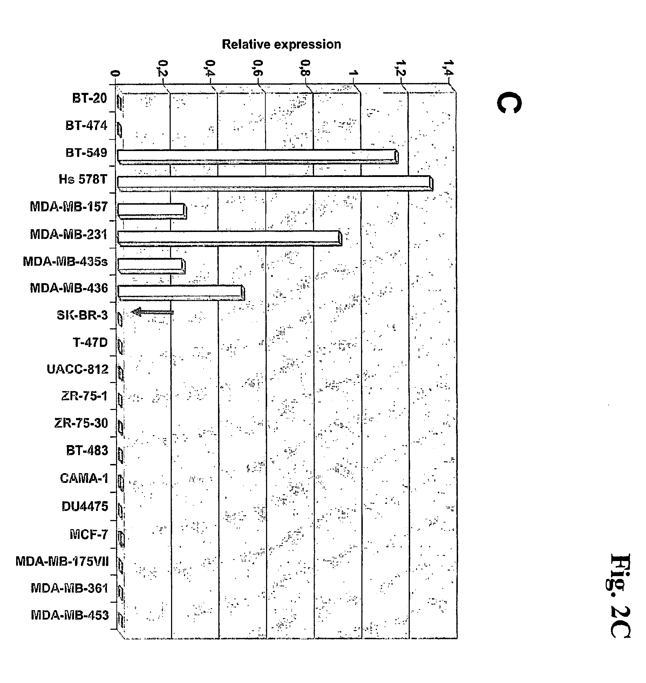

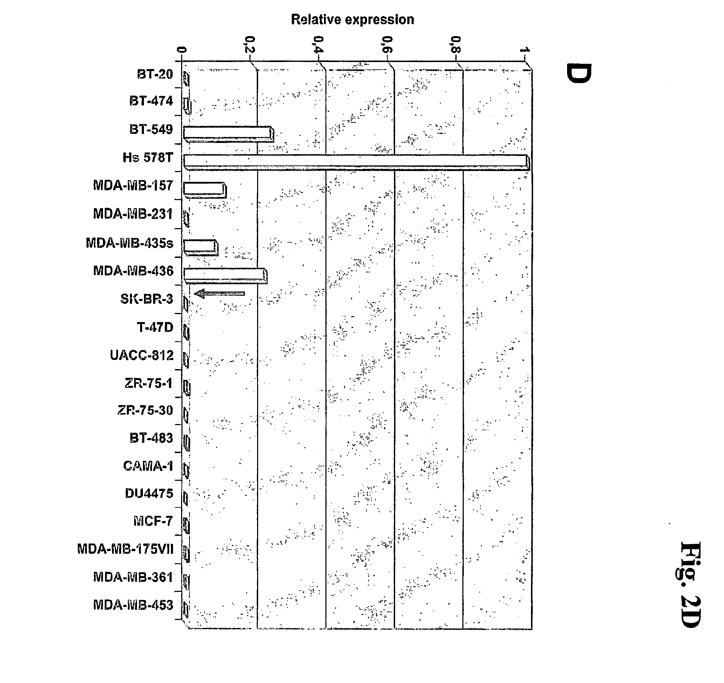

- Fig. 2: Assessment of steady-state levels of selected genes in an extended panel of invasive and non-invasive mammary carcinoma cell lines.
A: DDR2; B: PMP22; C: EMP3; D: N-cadherin mRNA steady-state levels of individual genes were determined by real-time RT-PCR as described in the Material and Methods section and normalized to 18S ribosomal RNA as an internal standard. Invasive cell lines are shown on the left-hand side of the diagrams separated by an arrow from the non-invasive cell lines.

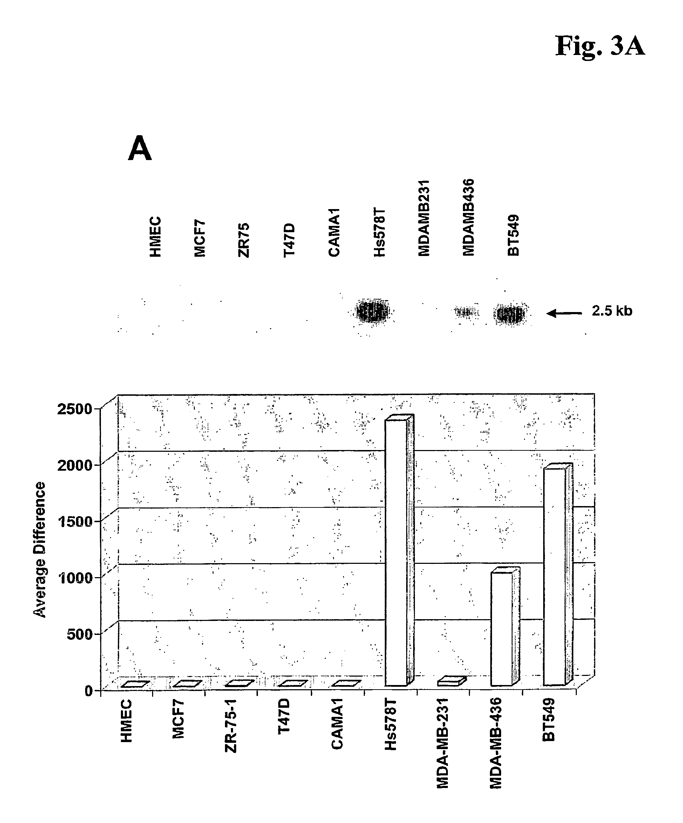

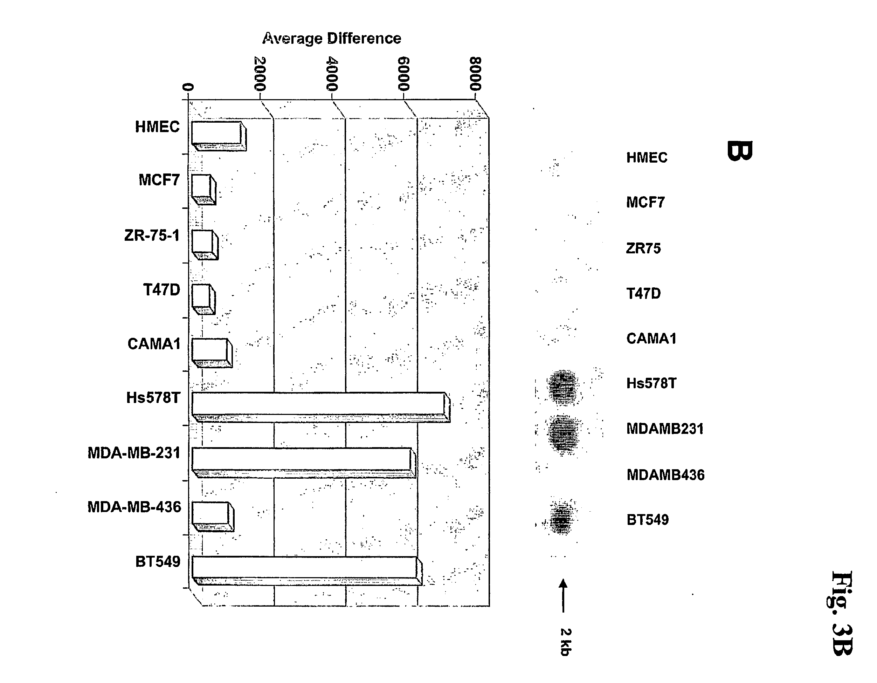

- Fig. 3: Deregulated expression of secretogranin II and CYR61 in four invasive and four non-invasive human mammary carcinoma cell lines.
A: secretogranin II; B: CYR61
Steady-state levels of mRNA were assessed by Northern blotting (above) and Affymetrix Technology (below).
Equal loading of the agarose gel slots for the Northern blot experiment was assessed by hybridization to a GAPDH probe (not shown).

### Example

### Introduction and Summary

The invasive status of several mammary carcinoma cell lines by their ability to penetrate into a collagen-fibroblast matrix was determined earlier and this property could be related with selected molecular characteristics. Cells with ER, PR and pS2, but no PAI-1 expression showed a non-invasive phenotype, while cells expressing PAI-1 mRNA, but no ER mRNA were scored as non-invasive (Tong, D., et al., Breast Cancer Res. Treat. 56 (1999) 91-97). In order to extend the panel of genes associated with the invasive phenotype, the Affymetrix GeneChip^{®} Technology (Coller, H.A., et al., Proc. Natl. Acad. Sci. USA 97 (2000) 3260-3265; De Lange, R., et al., Anticancer Res. 21 (2001) 2329-2339; Eisen, M.B., et al., Proc. Natl. Acad. Sci. USA 25 (1998) 14863-14868) to four non-invasive and four invasive mammary carcinoma cell lines for identification of the steady-state level of mRNA corresponding to 12625 genes. Expression of selected genes was subsequently investigated in an extended panel of mammary carcinoma cell lines and in mammary tumors. In the following it was focused on transmembrane receptors, secreted proteins and kinases according to their tractability as diagnostic markers and as targets for therapy.

### Material and Methods

### Cells and culture conditions

Human mammary epithelial cells (HMEC) were obtained from BioWhittaker, Inc (Walkersville, Maryland, USA) and cultured in MEGM^{®} (Mammary Epithelial Cell Medium).

The BT-20, BT-474, BT-549, Hs 578T, MDA-MB-157, MDA-MB-231, MDA-MB-435S, MDA-MB-436,SK-BR-3, T-47D, UACC-812, ZR-75-1, ZR-75-30, BT-483, CAMA-1, DU4475, MCF-7, MDA-MB-175-VII, MDA-MB-361 and MDA-MB-453 cell lines were obtained from the American Type Culture Collection (ATCC, Rockville, MD, USA) and cultured as recommended by ATCC. The cell lines have been classified as invasive and non-invasive on the basis of their migration into a collagen-fibroblast matrix (Tong, D., et al., Breast Cancer Res. Treat. 56 (1999) 91-97) (Table I). To determine the transcriptional profiles of the cell lines, cells were harvested at 70-80% confluency.

### cDNA array hybridization

Total RNA was isolated from 1x10⁷ cells using the Rneasy Mini Kit (Qiagen, Hilden, Germany). cRNA preparation and array hybridization were performed according to Affymetrix protocols. Briefly, double-stranded cDNA was synthesized from 10 µg total RNA by using cDNA Synthesis Kit (Roche Diagnostics, Mannheim, Germany) with a T7-(dT)₂₄ primer incorporating a T7 RNA polymerase promoter (5'GGCCAGTAATTGTAATACGACTCACTATGGGAGGC-GG-T₂₄-VN-3'). Double-stranded cDNA was purified with the modified High Pure RNA Tissue Kit (Roche Diagnostics, Mannheim, Germany). *In vitro* transcription was performed with cDNA to generate biotin-labeled cRNA with the MegaScript T7 Kit (Ambion Inc., Austin Texas, USA) as described by the manufacturer. The biotinylated cRNA was purified with the RNeasy Mini Kit (Qiagen), fragmented to 50-200 nucleotides by incubation at 94°C for 35 min in the presence of 200 mM Tris-acetate, pH 8.1, 500 mM potassium acetate and 150 mM magnesium acetate. 15 µg of the fragmented cRNA was hybridized for 16 h at 45°C to the U95Av2 Affymetrix array. After hybridization, the arrays were washed according to Affymetrix protocols and stained with streptavidin (Roche Diagnostics, Mannheim, Germany). This process was followed by incubation with biotinylated goat anti-streptavidin (Vector Laboratories Inc., Burlingame, CA, USA) and restaining with streptavidin-phycoerythrin (Molecular Probes Inc., Eug, USA) on the Affymetrix fluidics station. Arrays were finally scanned with an argon-ion laser confocal microscope (Hewlett-Packard G2500A Gene Array Scanner, Affymetrix). The intensity for each probe set of the array was assessed with Affymetrix Microarray Suite 4.0 software according to standard Affymetrix procedures.

### Data analysis

Roche Affymetrix Chip Experiment-Analyses (RACE-A) software was used to determine the relative abundance of each mRNA, based on the average difference (Avg Diff) representing the perfect match minus the mismatch for each gene-specific probe set. The average difference values for each target transcript on each array were normalized to the overall fluorescence intensity of the same array. The experiments were carried out in duplicate for each cell line. For identification of common differentially expressed genes in the initial set of cell lines, a fold change was calculated for each transcript between the cell line and the reference (HMEC).

In the case of an increase, the fold change is calculated as:
[avg_din_n_avg_cond2] / [avg_diff_n_avg_cond1-1].

In the case of a decrease the following formula was used: - [avg_diff_n_avg,cond1]/ [avg_diff_n_avg_cond2 +1]. Avg_diff n_avg is the average of normalized avg_diff of the duplicate experiments of a condition. Normalized average difference values of 80 or less were set to 80. This results in attenuation of the reported change in expression when comparison involves a transcript that is not expressed or is expressed at a very low level. Average-linkage hierarchical clustering was applied by using the CLUSTER program and the results were displayed by using TREEVIEW (Eisen, M.B., et al., Proc. Natl. Acad. Sci. USA 25 (1998) 14863-14868). For the cluster diagram presented in Fig. 1, the input parameters were set to select the subset of genes that had a fold change of two or higher in at least three or more cell lines. The level of gene expression was visualized using a color code varying from red (increased) to green (decreased) relative to the reference cell line (HMEC).

### Northern Blotting

Total RNA (10 µg) was electrophoresed through a 1% denaturing formaldehyde gel and blotted to BrightStar-Plus^{™} positively-charged nylon membranes (Ambion Inc., Austin, Texas, USA) by capillary downward transfer and fixed by exposure to ultraviolet light. Membranes were prehybridized for 30 min at 68°C with ExpressHyb hybridization solution (Clontech, Palo Alto, CA, USA). α-[³²P]dATP-labeled probes were prepared using the StripEZ^{™} DNA Kit (Ambion Inc., Austin, Texas, USA). Denatured probe was added to prewarmed hybridization solution to result in 2 x 10⁶ cpm/ml. Membranes were incubated with the probe solution with continuous rotation at 68°C for 12 h. After incubation with probe solution, membranes were washed three times with *solution I* (2× standard sodium citrate, 0.05% SDS) at room temperature, and then washed in *solution II* (0.1x standard sodium citrate 0.1% SDS) at 50°C. The membranes were then exposed to Cronex, Medical X-Ray Films (Sterling Diagnostic Imaging Inc., USA) at -80°C for 72 h. Blots then were stripped and rehybridized with a glyceraldehyde-3-phosphate dehydrogenase cDNA probe to confirm equal loading of the samples.

### RNA extraction and reverse transcription

Total RNA was extracted from cell lines using RNeasy^{®} Midi Kit (QIAGEN Inc., Hilden, Germany). Residual DNA was removed by treatment with DNAse I (QIAGEN Inc., Hilden, Germany). RNA quality and quantity were determined by UV spectrophotometry and agarose gel electrophoresis. 1µg RNA was reverse transcribed into first strand cDNA following the protocol of the First Strand cDNA Synthesis Kit (Roche Diagnostics, Penzberg, Germany) using random hexanucleotide primers. The reaction mixture was incubated at 25°C for 10 min followed by incubation at 42°C for 60 min and inactivation of the reverse transcriptase by heating at 99°C for 5 min and cooling at 4°C for 5 min.

### Real-time RT-PCR (LightCycler Technology)

Quantitative PCR was performed with the Roche LightCycler System (Roche Diagnostics, Mannheim, Germany). PCR reactions were carried out using the DNA Master SYBR^{®} Green I Kit (Roche Diagnostics, Mannheim, Germany) reaction mix. The PCR reactions were performed in a volume of 20 µl containing 2 µl of template cDNA, 2 µl LC DNA Master SYBR Green I mix and 5 pmol of each gene-specific primer (DDR2 forward primer 5-GAGTTCTGCAA-AAGCTCAGG-3; DDR2 reverse primer 5-TCAGGTCATCAGGTTCCACT-3; PMP22 forward primer 5-GAAGAAGGGGTTACGCTGTT-3; PMP22 reverse primer 5-CCTGAGGAAGA-GGTGCTACA-3; EMP3 forward primer 5-ACTTTGGACAAGTCCTGGTG-3; EMP3 reverse primer 5-AGAGCTGGAACATGAACAGG-3; N-cadherin forward primer 5-GAAGGATGTG-CATGAGGAC-3; N-cadherin reverse primer 5-TCCTGGGTCTCTTTGTCTTG-3) in a final concentration of 3 mM MgCl₂. For target amplification the cycling conditions were as follows: initial denaturation at 95°C for 1 min, followed by 40 cycles of denaturation at 95°C for 0 sec, annealing at 58°C for 5 sec and extension at 72°C for 8 sec. The amount of fluorescent product was measured in single-acquisition mode at 72°C after each cycle. To confirm amplification specificity, the PCR products were subjected to a melting curve analysis using the program run for 1 cycle at 95°C with 0 sec hold, 65 °C with 15 sec hold and 95°C with 0 sec hold at the step acquisition mode. To define the dynamic range and quantitative nature of real-time RT-PCR, standard curves for target and 18S rRNA were constructed using serial dilutions of total cDNA from cell line Hs578T. To ensure the fidelity of total RNA extraction and reverse transcription, all samples were subjected to real-time RT-PCR analysis with oligonucleotide primers (forward primer, 5-AGTTGGTGGAGCGATTTGTC-3; reverse primer, 5-GCTGAGCCAGTCAGTG-TAG-3) specific for 18S rRNA. Relative mRNA levels for defined genes were calculated in relation to the standard dilution series, and normalized to 18S rRNA values. Each sample was tested in duplicate.

### Summary of the results

### Cell lines and differentially expressed genes

Affymetrix profiling experiments were performed with four non-invasive and four invasive human mammary carcinoma cell lines. Non-invasive cell lines: MCF-7, ZR-75-1, T-47D and CAMA-1; invasive cell lines: Hs 578T, MDA-MB-231, MDA-MB-436 and BT-549. As shown in Table I, the non-invasive cell lines are ER⁺, PR⁺ and PAI-1⁻, the invasive cell lines score ER⁻, PR⁻, pS2⁻ and PAI-1⁺. The extended panel of cell lines used for further investigation is summarized in Table I.

**Table I (Tabulation of human mammary carcinoma cell lines used in this study. ER, PR, pS2 and PAI-1 status are shown. Invasiveness refers to migration of the cells into a collagen fibroblast matrix.):**

| | ER | PR | pS2 | PAI-1 | invasive status |
|---|---|---|---|---|---|
| BT20 | - | - | - | + | + |
| BT474 | + | + | + | - | + |
| BT549 | - | - | - | + | + |
| Hs578T | - | - | - | + | + |
| MDA-MB-157 | - | - | - | + | + |
| MDA-MB-231 | - | .. | - | + | + |
| MDA-MB-435 | - | - | - | - | + |
| MDA-MB-436 | - | - | - | + | + |
| SK-BR-3 | - | - | - | + | - |
| T47D | + | + | - | - | - |
| UACC-812 | + | - | + | + | - |
| ZR-7M | + | + | + | - | - |
| ZR-75-30 | + | - | + | - | - |
| BT483 | + | - | + | - | - |
| CAMA1 | + | + | + | - | - |
| DU4475 | - | - | - | - | - |
| MCF-7 | + | + | + | - | - |
| MDA-MB-175 | + | - | + | - | - |
| MDA-MB-361 | + | + | + | - | - |
| MDA-MB-453 | - | - | - | - | - |

The invasive properties and the status with respect to ER, PR, pS2 and PAI-1 are shown. The extended panel comprises 20 cell lines (8 invasive, 12 non-invasive). It is noteworthy that three of the non-invasive cell lines are ER⁻, whereas one of the invasive cell lines scored positive for ER. Furthermore, two of the non-invasive cell lines scored positive for PAI-1, two of the invasive cell lines were assessed as PAI-1 negative. The steady-state level of mRNA corresponding to 12625 genes with deregulated expression in all of the four invasive cell lines was investigated based on the following threshold levels: fold change of two or higher in at least three or more cell lines. The expression level of individual genes was calculated by defining the expression level of the corresponding gene in human mammary epithelial cells (HMEC) as a baseline. Based on the criteria as outlined above, 86 genes (0.7 %) were upregulated in the invasive cell lines and 321 genes (2.5 %) were downregulated in the invasive cell lines. In the following, genes are describe which are upregulated in the invasive cell lines and match with the categories transmembrane receptors, secreted proteins and kinases.

Based on the mRNA expression profiles, clustering based on the Eisen logarithm was performed as described in the Materials and Methods section. As shown in Fig. 1, the invasive and non-invasive cell lines cluster as separate entities.

### Transmembrane and GPI-linked receptors

18 transmembrane receptors were identified upregulated in the invasive cell lines. Deregulated expression of transmembrane tyrosine kinase receptors is documented for mammary carcinoma. In addition to documented EGFR, DDR2 (Vogel, W., et al., Mol. Cell. 1 (1997) 13-23), EphA2 and axl were identified, receptors which have not been noted before in the context of mammary carcinoma metastasis. EphA2 overexpression was shown to cause tumorigenesis of mammary epithelial cells (Zelinski, D.P., et al., Cancer Res. 61 (2001) 2301-2306) and axl was shown to be overexpressed in metastatic colon cancer (Craven, R.J., et al., Int. J. Cancer 60 (1995) 791-797). The role of GPI-linked urokinase receptor (uPAR) as a prognostic marker for mammary carcinoma has been established before (Guyton, D.P., et al., Breast J. 6 (2000) 130-136). Also CD44 has been implicated in mediating metastasis in several systems (Herrlich, P., et al., Immunol. Today 14 (1993) 395-399). In the category of proteases aminopeptidase N was identified as a putative key mediator of invasion. Furthermore, expression of aminopeptidase N was correlated with drug resistance in breast carcinomas (Dixon, J., et al., J. Clin. Pathol. 47 (1994) 43-47).

Three 4TM proteins were found to be overexpressed in the invasive cell lines: PMP22, EMP1 and EMP3. PMP22 was shown to be overexpressed in glioma and osteogenic sarcoma (Huhne, K., et al., J. Neurosci. Res. 58 (1999) 624-631), whereas EMP3 was found to be overexpressed in mammary carcinoma cell line MCF-7_{ADR} which is estradiol-independent for growth, estrogen-receptor negative, tamoxifen resistant, vimentin positive, adriamycin resistant and invasive *in vitro* and *in vivo* compared with cell line MCF-7, which exhibits inverse characteristics (Ben-Porath, I., and Benvenisty, N., Gene 183 (1996) 69-75; Schiemann, S., Anticancer Res. 17 (1997) 13-20). CD97 is abundantly expressed in cells of hematopoietic origin and has been described as a dedifferentiation marker in thyroid carcinoma (Aust, G., et al., Cancer Res. 57 (1997) 1798-1806). L6 surface antigen is highly expressed in lung, breast, colon and ovarian carcinomas and has attracted attention as a target for antibody-based treatments (Marken, J.S., et al., Proc. Natl. Acad. Sci. USA 89 (1992) 3503-3507). Immunoglobulin superfamily molecule ICAM1 (intercellular adhesion molecule 1) binds to integrin LFA1 and promotes adhesion. N-cadherin was shown to represent a path-finding cadherin allowing cellular invasion and migration in both normal and pathophysiological processes (Zeitvogel, A., et al., Am. J. Pathol. 159 (2001) 1839-1852).

Expression of DDR2, PMP22, EMP3 and N-cadherin at the RNA level was determined in the extended panel of 20 mammary carcinoma cell lines (Fig. 2). For these genes, consistently very low levels of mRNA were observed in the non-invasive cell lines. DDR2 (Fig. 2A), PMP22 (Fig. 2B), EMP3 (Fig. 2C) and N-cadherin (Fig. 2D) mRNA was significantly overexpressed in the invasive cell lines.

### Secreted proteins

In the category secreted proteins 18 mRNAs were identified encoding secreted proteins with increased steady-state level in the invasive cell lines. In the following selected proteins are commented. Expression of urokinase was correlated with poor prognosis in patients with mammary carcinoma (Duffy, M.J., Clin. Chem. 48 (2002) 1194-1197). Plasminogen activator inhibitor 1 (PAI-1) was also identified as a marker for adverse prognosis in patients with mammary carcinoma (Bajou, K., et al., Int. J. Cancer 100 (2002) 501-506) and shown to be involved in mediating angiogenesis (Werner, H., and Le Roith, D., Crit. Rev. Oncog. 8 (1997) 71-92). mRNAs for insulin-like growth factor binding proteins (IGFBP3, IGFBP6, IGFBP7) are overexpressed in the invasive cell lines. Some of these factors seem to exert physiological effects that are not dependent on interaction with insulin-like growth factor (IGF) (Werner, H., and Le Roith, D., Crit. Rev. Oncog. 8 (1997) 71-92). Il8 was shown to induce proliferation, migration and invasion of endothelial cells and hence neovascularization (Gutman, M., et al., Cancer Res. 55 (1995) 2470-2475). Pleiotrophin (PTN) can serve as an angiogenesis factor (Czubyko, F., et al., Breast Cancer Res. Treat. 36 (1995) 157-168) and exhibits high levels of expression in 60 % of tumor samples from breast cancer patients (Riegel, A.T., and Wellstein, A., Breast Cancer Res. Treat. 31 (1994) 309-314). Cyr61 is a cysteine-rich, estrogen-inducible angiogenic factor which is overexpressed in breast cancer and is associated with more advanced disease (Xie, D., et al., J. Biol. Chem. 276 (2001) 14187-14184). Secretogranin II (Chromogranin C) is located within secretory granules of a variety of endocrine cells and neurons and is a member of the chromogranin (secretogranin) secretory protein family. Expression has been documented in spontaneous mammary tumors in aging Fischer-344 rats (Umemura, S., et al., Pathol. Int. 51 (2001) 667-670). Other identified deregulated genes such as tissue inhibitor of metalloproteinases 1 (TIMP1), CRIM1, interleukin 6, aldehyde reductase 1, nerve growth factor (NGF), lysyl-oxidase-like 2 (LOXL2), small inducible cytokine A2 (SCA2) and tumor necrosis factor alpha-induced protein 6 (TNFAIP6) deserve further investigation. Expression of mRNAs encoding two secreted proteins, secretogranin II (Fig. 3A) and CYR61 (Fig. 3B) was analysed by comparing Northern blotting with Affymetrix Technology. The results of these methods match very well and secretogranin II as well as CYR61 were found to be overexpressed in three of the invasive cell lines.

### Kinases

Five kinases were identified with increased steady-state levels in the invasive cell lines. RAGE is a serine-threonine kinase and member of the MAP kinase superfamily (Miyata, Y., et al., Genes Cells 4 (1999) 299-309). STK17A is a serine-threonine kinase which seems to be involved in apoptotic signaling (Sanjo, H., et al., J. Biol. Chem. 273 (1998) 29066-29071). Dyrk3 is a dual specifity protein kinase implicated in cellular growth (Becker, W., et al., J. Biol. Chem. 273 (1998) 25893-25902). Mitogen-activated protein kinase kinase kinase kinase 4 (MAP4K4) is a serine-threonine kinase which activates c-Jun N-terminal kinase (JNK) signaling and other specific signaling pathways (Xue, Y., et al., Development 128 (2001) 1559-1572). None of these kinases has previously been associated with mammary carcinoma.

### Discussion of the results

Transcriptional profiling experiments using Affymetrix GeneChip^{®} Technology with four invasive versus four non-invasive human mammary carcinoma cell lines has revealed that the invasive and the non-invasive cell lines cluster as separate entities. As baseline the steady-state level of individual mRNAs in human mammary epithelial cells (HMEC) was have used. Based on the scoring criteria outlined in the Materials and Methods section, it was found that out of 12625 genes investigated, 86 genes (0.7 %) were upregulated and 321 genes (2.5 %) were downregulated in at least three of the invasive cell lines compared with at least three of the non-invasive cell lines. In accordance with the criteria for biological and chemical tractability as targets for diagnosis and treatment, transmembrane receptors, secreted proteins and kinases were further investigated. Relative mRNA expression of DDR2, PMP22, EMP3 and N-cadherin was investigated in an extended panel of cell lines, all characterized with respect to ER, PR, pS2 and PAI-1 expression as well as invasiveness by their ability to penetrate into a collagen-fibroblast matrix (Table I). 18 transmembrane receptors, 18 secreted proteins and 5 kinases were identified with increased steady-state mRNA level in the invasive cell lines.

In the category "secreted proteins" several proteins were identified which have already been described as prognostic markers or putative targets for treatment of mammary carcinoma, such as urokinase (uPA) (Duffy, M.J., Clin. Chem. 48 (2002) 1194-1197) and plasminogen activator inhibitor 1 (PAI-1) (Zeitvogel, A., et al., Am. J. Pathol. 159 (2001) 1839-1852; Duffy, M.J., Clin. Chem. 48 (2002) 1194-1197) or mediators of angiogenesis such as Il8 (Werner, H., and Le Roith, D., Crit. Rev. Oncog. 8 (1997) 71-92), pleiotrophin (PTN) (Gutman, M., et al., Cancer Res. 55 (1995) 2470-2475) and CYR61 (Riegel, A.T., and Wellstein, A., Breast Cancer Res. Treat. 31 (1994) 309-314). The other secreted proteins identified, such as tissue inhibitor of metalloproteinases 1 (TIMP1), CRIM1, interleukin 6, IGFBP3, IGFBP6, IGFBP7, nerve growth factor (NGF), secretogranin II, lysyloxidase-like 2 (LOXL2), small inducible cytokine A2 (SCA2) and tumor necrosis factor alpha-induced protein 6 (TNFAIP6), deserve further investigation. The steady-state levels of mRNA for secretogranin II and CYR61 were determined by Northern blotting (Fig. 3). The data demonstrate the correspondence of RNA levels assessed by Northern blotting and Affymetrix analysis of secretogranin II and CYR61.

The five kinases (4 serine-threonine kinases, one dual-specificity protein kinase) are all new in the context of mammary carcinoma progression. Investigation of deregulation of RAGE (Umemura, S., et al., Pathol. Int. 51 (2001) 667-670), STK17A (Miyata, Y., et al., Genes Cells 4 (1999) 299-309), DIRK3 (Sanjo, H., et al., J. Biol. Chem. 273 (1998) 29066-29071), MAP4K4 (Becker, W., et al., J. Biol. Chem. 273 (1998) 25893-25902) and PFTK1 in the extended panel of cell lines and in mammary carcinomas with different staging deserves further interest.

In the category "transmembrane receptors" several mRNAs were identified encoding for gene products with known implications in breast cancer progression in the invasive cell lines, such as urokinase receptor (uPAR) (Craven, R.J., et al., Int. J. Cancer 60 (1995) 791-797), EGFR, ephrin receptor EphA2, axl receptor tyrosine kinase and L6 antigen. It was focused on four genes which have not been previously described in this context: transmembrane tyrosine kinase DDR2, 4TM receptors PMP22 and EMP3 and adhesion molecule N-cadherin. Only very low levels of mRNA for these genes were observed in the non-invasive cell lines, whereas significantly increased mRNA levels were observed in the majority of the invasive cell lines (Fig. 2). These findings unequivocally indicate that the correlation of genes deregulated originally determined in a panel of four invasive and four non-invasive mammary carcinoma cell lines can be extrapolated to an extended panel of invasive and non-invasive mammary carcinoma cell lines, probably also for other genes as shown in Table II (Selected categories of genes with increased expression in invasive versus non-invasive human mammary carcinoma cell lines. A: Transmembrane and GPI-linked receptors; B: Secreted proteins; C: Kinases Transcriptional profiling of the cell lines as referred to above, selection of deregulated genes acording to the scoring criteria and clustering were performed as described in the Example - Materials and Methods section. Red coloured bars indicate upregulated genes, green coloured bars show downregulated genes. The transcriptional profile of HMEC was used as a baseline.):

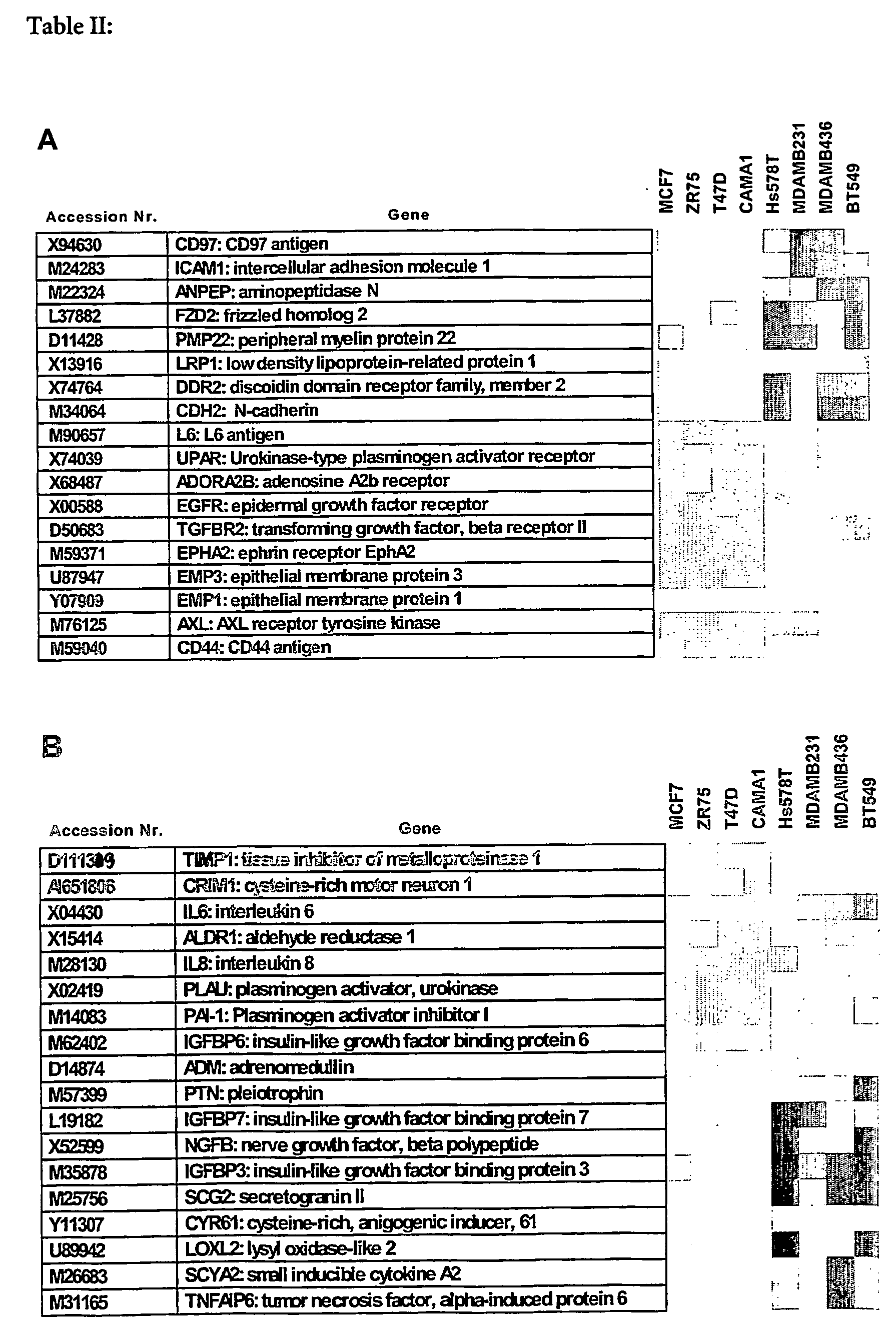

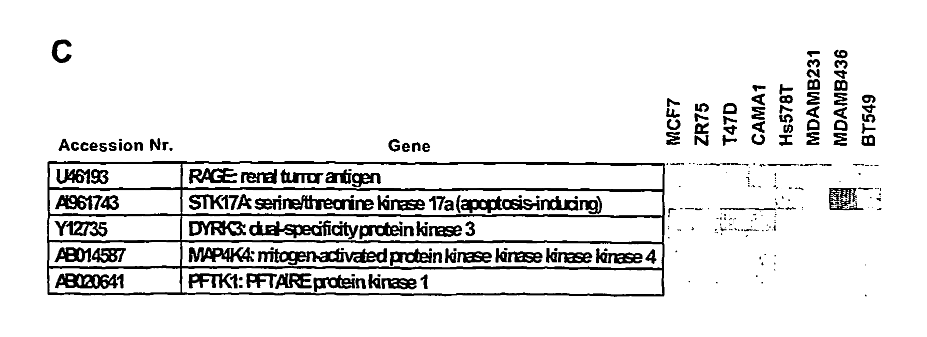

### List of References

Ahr, A., et al., Lancet 359 (2002) 131-132
Altschul, S.F., et al., J. Mol. Biol. 215 (1990) 403-410
Altschul, S.F., et al., Nucleic Acids Res. 25 (1997) 3389-3402
Amann, E., et al., Gene 69 (1988) 301-315
Arkin, A.P., and Youvan, D.C., Proc. Natl. Acad. Sci. USA 89 (1992) 7811-7815
Arnon et al., "Monoclonal Antibodies For Immunotargeting Of Drugs In Cancer Therapy", In: Monoclonal Antibodies And Cancer Therapy, Reisfeld et al. (eds.), Alan R. Liss, Inc., 1985, pp. 243-256
Aust, G., et al., Cancer Res. 57 (1997) 1798-1806
Ausubel et al., ed., Current Protocols in Molecular Biology, John Wiley & Sons, New York, 1987-1999
Bajou, K., et al., Int. J. Cancer 100 (2002) 501-506
Baldari, C., et al., EMBO J. 6 (1987) 229-234
Baldwin et al. (eds.), "Analysis, Results, And Future Prospective Of The Therapeutic Use Of Radiolabeled Antibody In Cancer Therapy", In: Monoclonal Antibodies For Cancer Detection And Therapy, Academic Press, 1985, pp. 303-316
Banerji, J., et al., Cell 33 (1983) 729-740
Barany, F., Proc. Natl. Acad. Sci. USA 88 (1991) 189-193
Bartel, D.P., and Szostak, J.W., Science 261 (1993) 1411-1418
Bartel, P., et al., Biotechniques 14 (1993) 920-924
Becker, W., et al., J. Biol. Chem. 273 (1998) 25893-25902
Beidler, C.B., et al., J. Immunol. 141 (1988) 4053-4060
Ben-Porath, I., and Benvenisty, N., Gene 183 (1996) 69-75
Better, M., et al., Science 240 (1988) 1041-1043
Bradley, A., Curr. Opin. Biotechnol. 2 (1991) 823-829
Bradley, Teratocarcinomas and Embryonic Stem Cells, A Practical Approach, Robertson, ed., IRL, Oxford, 1987, pp. 113-152
Breslauer, K.J., et al., Proc. Natl. Acad. Sci. USA 83 (1986) 3746-3750
Byrne, G.W., and Ruddle, F.H., Proc. Natl. Acad. Sci. USA 86 (1989) 5473-5477
Calame, K., and Eaton, S., Adv. Immunol. 43 (1988) 235-275
Camper, S.A., and Tilghman, S.M., Genes Dev. 3 (1989) 537-546
Carell et al., Angew. Chem. Int. Ed. Engl. 33 (1994) 2061
Carrell et al., Angew. Chem. Int. Ed. Erg. 33 (1994) 2059
Chen, S.H., et al., Proc. Natl. Acad. Sci. USA 91 (1994) 3054-3057
Cho, C.Y., et al., Science 261 (1993) 1303-1305
Cole et al., In: Monoclonal Antibodies and Cancer Therapy, Alan R. Liss, Inc., 1985, pp. 77-96
Coligan et al., ed., Current Protocols in Immunology, John Wiley & Sons, New York, 1994
Coller, H.A., et al., Proc. Natl. Acad. Sci. USA 97 (2000) 3260-3265
Craven, R.J., et al., Int. J. Cancer 60 (1995) 791-797
Cruikshank, W.W., et al., J. Acquired Immune Deficiency Syndromes and Human Retrovirology 14 (1997) 193-203
Cull, M.G., et al., Proc. Natl. Acad. Sci. USA 89 (1992) 1865-1869 Current Protocols in Molecular Biology, John Wiley & Sons, N.Y.,1989, sections 6.3.1 -6.3.6
Cwirla, S.E., et al., Proc. Natl. Acad. Sci. 87 (1990) 6378-6382
Czubyko, F., et al., Breast Cancer Res. Treat. 36 (1995) 157-168
De Lange, R., et al., Anticancer Res. 21 (2001) 2329-2339
Delagrave, S., et al., Prot. Eng. 6 (1993) 327-331
Devlin, J.J., et al., Science 249 (1990) 404-406
DeWitt, S.H., et al., Proc. Natl. Acad. Sci. USA 90 (1993) 6909-6913
Dixon, J., et al., J. Clin. Pathol. 47 (1994) 43-47
Duffy, M.J., Clin. Chem. 48 (2002) 1194-1197
Edlund, T., et al., Science 230 (1985) 912-916
Eisen, M.B., et al., Proc. Natl. Acad. Sci. USA 25 (1998) 14863-14868
EP-A 0 125 023
EP-A 0 171496
EP-A 0 173 494
EP-A 0 184 187
EP-A 0 264 166
EP-A 0 476 014
Erb, E., et al., Proc. Natl. Acad. Sci. USA 91 (1994) 11422-11426
Felici, F., et al., Mol. Biol. 222 (1991) 301-310
Finn, P.J., et al., Nucleic Acids Res. 24 (1996) 3357-3363
Fishel, R., et al., Science 266 (1994) 1403-1405
Fodor, S.P., et al., Nature 364 (1993) 555-556
Fuchs, P., et al. Bio/Technology 9 ((1991)) 1369-1372
Gallop, M.A., et al., J. Med. Chem. 37 (1994) 1233-1251
Gautier, C., et al., Nucleic Acids Res. 15 (1987) 6625-6641
Goeddel, Methods in Enzymology: Gene Expression Technology, Vol. 185, Academic Press, San Diego, CA,1991
Golub, T.R., et al., Science 286 (1999) 531-537
Gottesman, In: Gene Expression Technology: Methods in Enzymology, Vol. 185, Academic Pres, San Diego, CA, 1990, pp. 119-128
Griffiths, A.D., et al., EMBO 12 (1993) 725-734
Guatelli, J.C., et al., Proc. Natl. Acad. Sci. USA 87 (1990) 1874-1878
Gutman, M., et al., Cancer Res. 55 (1995) 2470-2475
Guyton, D.P., et al., Breast J. 6 (2000) 130-136
Hage, D.S., and Tweed, S.A., J. Chromatogr. B. Biomed. Sci. Appl. 699 (1997) 499-525
Harlow and Lane, Antibodies: Laboratory Manual, Cold Spring Harbor Laboratory Press, Cold Spring Harbor, New York, 1988
Haseloff, J., and Gerlach, W.L., Nature 334 (1988) 585-591
Hay, B.N., et al., Hum. Antibod. Hybridomas 3 (1992) 81-85
Heegaard, N.H., J. Mol. Recognit. 11 (1998) 141-148
Helene, C., Anticancer Drug Des. 6 (1991) 569-584
Helene, C., et al., Ann. N. Y. Acad. Sci. 660 (1992) 27-36
Hellstrom et al., "Antibodies For Drug Delivery", In: Controlled Drug Delivery, 2nd ed., Robinson et al. (eds.), Marcel Dekker, Inc., 1987, pp. 623-653
Herrlich, P., et al., Immunol. Today 14 (1993) 395-399
Hesketh, In: The Oncogene and Tumour Suppressor Gene Facts Book, 2nd ed., Academic Press, 1997
Hogan, Manipulating the Mouse Embryo, Cold Spring Harbor Laboratory Press, Cold Spring Harbor, N.Y.,1986
Houghten, R.A., et al., Biotechniques 13 (1992) 412-421
Huhne, K., et al., J. Neurosci. Res. 58 (1999) 624-631
Huse, W.D., et al., Science 246 (1989) 1275-1281
Hyrup, B., and Nielsen, P.E., Bioorg. Med. Chem. 4 (1996) 5-23
Iizuka, N., Cancer Res. 62 (2002) 3939-3944
Ike, Y., et al., Nucleic Acid Res. 11 (1983) 477-488
Inoue, H., et al., FEBS Lett. 215 (1987) 327-330
Inoue, H., et al., Nucleic Acids Res. 15 (1987) 6131-6148
Itakura, K., et al., Annu. Rev. Biochem. 53 (1984) 323-356
Itakura, K., et al., Science 198 (1977) 1056-1063
Iwabuchi, K., et al., Oncogene 8 (1993) 1693-1696
Jespers, L.S., et al., Bio/Technology 12 (1994) 899-903
Jones, P.T., et al., Nature 321 (1986) 522-525
Karlin, S., and Altschul, S.F., Proc. Natl. Acad. Sci. USA 87 (1990) 2264-2268
Karlin, S., and Altschul, S.F., Proc. Natl. Acad. Sci. USA 90 (1993) 5873-5877
Kaufman, R.J., et al., EMBO J. 6 (1987) 187-193
Kessel, M., and Gruss, P., Science 249 (1990) 374-379
Kohler, G., and Milstein, C., Nature 256 (1975) 495-497
Kozbor et al., Immunol. Today 4 (1983) 72
Kurjan, J., and Herskowitz, I., Cell 30 (1982) 933-943
Kwoh, D.Y., et al., Proc. Natl. Acad. Sci. USA 86 (1989) 1173-1177
Lakso, M., et al. Proc. Natl. Acad. Sci. USA 89 (1992) 6232-6236
Lam, K.S., Anticancer Drug Des. 12 (1997)145-167
Lam, K.S., et al., Nature 354 (1991) 82-84
Lemaitre, M., et al., Proc. Natl. Acad. Sci. USA 84 (1987) 648-652
Letsinger, R.L., et al., Proc. Natl. Acad.Sci. USA 86 (1989) 6553-6556
Li, E., et al., Cell 69 (1992) 915-926
Linder, M.W., et al., Clin. Chem. 43 (1997) 254-266
Liu, A.Y., et al., J. Immunol. 139 (1987) 3521-3526
Liu, A.Y., et al., Proc. Natl. Acad. Sci. USA 84 (1987) 3439-3443
Lizard et al., Bio/Technology 6 (1988) 1197
Lockhart, D.J., et al., Nat. Biotechnol. 14 (1996) 1675-1680
Lonberg, N., and Huszar, D., Int. Rev. Immunol. 13 (1995) 65-93
Luckow, V.A., and Summers, M.D., Virology 170 (1989) 31-39
Madura, K., et al., J. Biol. Chem. 268 (1993) 12046-12054
Mag, M., and Engels, J.W., Nucleic Acids Res. 17 (1989) 5973-5988
Maher, L.J. 3rd., Bioassays 14 (1992) 807-815
Marken, J.S., et al., Proc. Natl. Acad. Sci. USA 89 (1992) 3503-3507
McGraw, R.A., et al., BioTechniques 8 (1990) 674-678
Milner, N., et al., Nat. Biotechnol. 15 (1997) 537-541
Miyata, Y., et al., Genes Cells 4 (1999) 299-309
Morrison, S.L., Science 229 (1985) 1202-1207
Myers and Miller, (1988)
Narang, Tetrahedron 39 (1983) 3
Nishimura, Y., et al., Cancer Res. 47 (1987) 999-1005
O'Gorman, S., et al., Science 251 (1991) 1351-1355
Oi et al., Bio/Techniques 4 (1986) 214
Perry-O'Keefe, H., et al., Proc. Natl. Acad. Sci. USA 93 (1996) 14670-14675
Peterser et al., Bioorganic Med. Chem. Lett. 5 (1975) 1119-11124
Pinkert, C.A., et al., Genes Dev. 1 (1987) 268-277
Polyak, K., et al., Nature 389 (1997) 300-305
Queen, C., and Baltimore, D., Cell 33 (1983) 741-748
Ramaswamy, S., et al., Nat. Genet. 33 (2003) 49-54
Riegel, A.T., and Wellstein, A., Breast Cancer Res. Treat. 31 (1994) 309-314
Rivas, G., and Minton, A.P., Trends Biochem Sci. 18 (1993 ) 284-287
Ruppert, J.M., et al., Mol. Cell. Bio. 8 (1988) 3104-3113
Sambrook et al., Molecular Cloning: A Laboratory Manual, 2nd ed., Cold Spring Harbor Laboratory Press, Cold Spring Harbor, NY, 1989
Sanjo, H., et al., J. Biol. Chem. 273 (1998) 29066-29071
Schiemann, S., Anticancer Res. 17 (1997) 13-20
Schultz, L.D., et al., Gene 54 (1987) 113-123
Scott, J.K., and Smith, G.P., Science 249 (1990) 386-390
Seed, B., Nature 329 (1987) 840-842
Shaw, D.R., et al., Natl. Cancer Inst. 80 (1988) 1553-1559
Sjolander, S. and Urbaniczky, C., Anal. Chem. 63 (1991) 2338-2345
Smith, D.B., and Johnson, K.S., Gene 67 (1988) 31-40
Smith, G.E., et al., Mol. Cell Biol. 3 (1983) 2156-2165
Studier et al., In: Gene Expression Technology: Methods in Enzymology, Vol. 85, Academic Press, San Diego, CA, 1991, pp. 60-89
Sun, L.K., et al., Proc. Natl. Acad. Sci. USA 84 ((1987)) 214-218
Szabo, A., et al., Curr. Opin. Struct. Biol. 5 (1995) 699-705
Thomas, K.R., and Capecchi, M.R., Cell 51 (1987) 503-512
Thorpe, "Antibody Carriers Of Cytotoxic Agents In Cancer Therapy: A Review", In: Monoclonal Antibodies '84: Biological And Clinical Applications, Pinchera et al. (eds.), 1985, pp. 475-506
Thorpe, P.E., and Ross, W.C., Immunol. Rev. 62 (1982) 119-158
Tong, D., et al., Breast Cancer Res. Treat. 56 (1999) 91-97
Umemura, S., et al., Pathol. Int. 51 (2001) 667-670
US 5,328,470
US 5,625,126
US 5,876,930
US 4,423,145
US 4,522,811
US 4,676,980
US 4,683,202
US 4,736,866
US 4,808,532
US 4,816,397
US 4,816,567
US 4,843,155
US 4,868,103
US 4,870,009
US 4,873,191
US 4,873,316
US 4,987,071
US 4,996,143
US 5,002,867
US 5,116,742
US 5,143,854
US 5,202,231
US 5,210,015
US 5,223,409
US 5,225,539
US 5,283,317
US 5,487,972
US 5,545,806
US 5,565,322
US 5,569,825
US 5,585,089
US 5,631,169
US 5,633,425
US 5,661,016
US 5,804,375
US 5,849,489
US 5,854,033
US 6,022,963
US 6,156,501
US 6,162,603
US 6,174,670
van der Krol, A.R., et al., Bio/Techniques 6 (1988) 958-976
van Ness, J., and Chen, L., Nucleic Acids Res. 19 (1991) 5143-5151
Velculescu, V.E., et al., Science 270 (1995) 484-487
Verhoeyen, M., et al., Science 239 (1988) 1534-1536
Vogel, W., et al., Mol. Cell. 1 (1997) 13-23
Wada, K., et al., Nucleic Acids Res. 20 (1992) 2111-2118
Weintraub et al., 1986, Trends in Genetics, Vol. 1(1)
Werner, H., and Le Roith, D., Crit. Rev. Oncog. 8 (1997) 71-92 Wigle, D.A., et al., Cancer Res. 62 (2002) 3005-3008
Wilmut, I., et al., Nature 385 (1997) 810-813
Winoto, A., and Baltimore, D., EMBO J. 8 (1989) 729-733
WO 86/01533
WO 87/02671
WO 88/09810
WO 89/10134
WO 89/10977
WO 89/11548
WO 90/02809
WO 90/11354
WO 90/15070
WO 91/01140
WO 91/17271
WO 92/01047
WO 92/0968
WO 92/09690
WO 92/10092
WO 92/15679
WO 92/18619
WO 92/20791
WO 93/01288
WO 93/04169
WO 93/17126
WO 94/10300
WO 97/07668
WO 97/07669
Wood, C.R., et al., Nature 314 (1985) 446-449
Worsham, M.J., et al.. (Cytometry 47 (2002) 56-59
Xie, D., et al., J. Biol. Chem. 276 (2001) 14187-14184
Xue, Y., et al., Development 128 (2001) 1559-1572
Zeitvogel, A., et al., Am. J. Pathol. 159 (2001) 1839-1852
Zelinski, D.P., et al., Cancer Res. 61 (2001) 2301-2306
Zervos, A.S., et al., Cell 72 (1993) 223-232
Zon, G., Pharm. Res. 5 (1988) 539-549
Zuckermann, R.N., et al., J. Med. Chem. 37 (1994) 2678-2685

## Claims

1. A method of determining whether or not a human mammary cancer cell containing patient sample has potential for tumor progression, the method comprising comparing:
a) the level of expression of marker genes in the patient sample, and
b) the normal level of expression of said marker genes in a sample from a control subject not afflicted with mammary cancer or the normal level of expression of said marker genes in a sample containing cells from a human mammary epithelial cell line,
wherein said marker genes comprise the group of marker genes consisting of the genes with a nucleic acid sequence according to SEQ ID NO: 1, 2,3 and 4 and a significant difference between the level of expression of said marker genes in the patient sample and the normal level of said marker genes in the sample from a control subject not afflicted with mammary cancer or in the sample containing cells from a human mammary epithelial cell line is an indication that the patient sample has potential for tumor progression.

2. The method according to claim 1, wherein said significant difference comprises an at least two fold difference between the level of expression of said marker genes in the patient sample and the normal level of expression of the same marker genes in the sample from the control subject or in the sample containing cells from a human mammary epithelial cell line.

3. The method according to claim 1, wherein said significant difference comprises an at least five fold difference between the level of expression of said marker genes in the patient sample and the normal level of expression of the same marker genes in the sample from the control subject or in the sample containing cells from a human mammary epithelial cell line.

4. The method according to any of the claims 1 to 3, wherein the sample is a breast tissue sample or a breast-associated fluid.

5. The method according to any of the claims 1 to 4, wherein the level of expression of said marker genes in the sample is assessed by detecting the presence in the sample of a protein encoded by each of said marker gene or a polypeptide or protein fragment comprising said protein.

6. The method of claim 5, wherein the presence of said protein, polypeptide or protein fragment is detected using a reagent which specifically binds with said protein, polypeptide or protein fragment.

7. The method of claim 6, wherein the reagent is selected from the group consisting of an antibody, an antibody derivative, and an antibody fragment.

8. The method of claim according to any of the claims 1 to 4, wherein the level of expression of said marker genes in the sample is assessed by detecting the presence in the sample of a transcribed polynucleotide encoded by each of said marker genes or a portion of said transcribed polynucleotide.

9. The method of claim 8, wherein the transcribed polynucleotide is a cDNA, mRNA or hnRNA.

10. The method according to any of the claims 8 to 9, wherein the step of detecting further comprises amplifying the transcribed polynucleotide.

11. The method according to any of the claims 1 to 4, wherein the level of expression of said marker genes in the samples is assessed by detecting the presence in the samples of a transcribed polynucleotide which anneals with each of said marker genes or anneals with a portion of said transcribed polynucleotide, under stringent hybridization conditions.

12. The use of a kit for assessing whether a patient has a risk of progression of breast cancer, wherein the kit comprises reagents for assessing expression of one or several marker genes, and said marker genes are comprising the group consisting of the genes with a nucleic acid sequence according to SEQ ID NO: 1,2, 3 and 4.

## Patentansprüche

1. Verfahren zur Bestimmung ob eine Probe enthaltend eine menschliche Brustkrebszelle Potential zur Tumorprogression aufweist, welches den Vergleich:
a) des Expressionsniveaus eines Markergens in der Patientenprobe, und
b) des normalen Expressionsniveaus des Markergens in einer Probe eines Kontrollsubjekts, welches nicht an Brustkrebs leidet, oder des normalen Expressionsniveaus des Markergens in einer Probe, die Zellen einer humanen Brust-Epithelzellinie enthält, umfasst,
wobei die Markergene die Gruppe der Markergene enthalten, die aus Genen mit der Nukleinsäuresequenz gemäß SEQ ID NO: 1, 2, 3 und 4 bestehen, und ein signifikanter Unterschied zwischen dem Expressionsniveau des Markergens in der Patientenprobe und dem normalen Niveau des Markergens in der Probe des Kontrollsubjekts, welches nicht an Brustkrebs leidet, oder in der Probe, die Zellen einer humanen Brust-Epithelzelllinie enthält, ein Hinweis darauf ist, dass die Patientenprobe das Potential zur Tumorprogression aufweist.

2. Verfahren gemäß Anspruch 1, wobei der signifikante Unterschied einen mindestens zweifachen Unterschied zwischen dem Expressionsniveau der Markergene in der Patientenprobe und dem normalen Expressionsniveau derselben Markergene in der Probe des Kontrollsubjekts oder in der Probe, die Zellen einer humanen Brust- Epithelzelllinie enthält, umfasst.

3. Verfahren gemäß Anspruch 1, wobei der signifikante Unterschied einen mindestens fünffachen Unterschied zwischen dem Expressionsniveau der Markergene in der Patientenprobe und dem normalen Expressionsniveau derselben Markergene in der Probe des Kontrollsubjekts oder in der Probe, die Zellen einer humanen Brust-Epithelzelllinie enthält, umfasst.

4. Verfahren nach einem der Ansprüche 1 bis 3, wobei die Probe eine Brustgewebsprobe oder eine Brust-assoziierte Flüssigkeit ist.

5. Verfahren nach einem der Ansprüche 1 bis 4, wobei das Expressionsniveau der Markergene in der Probe nachgewiesen wird durch Bestimmung der Anwesenheit eines Proteins in der Probe, welches durch jedes der Markergene kodiert wird, oder eines Polypeptids oder Proteinfragments enthaltend das Protein.

6. Verfahren nach Anspruch 5, wobei die Anwesenheit des Proteins, Polypeptids oder Proteinfragmentes unter Verwendung eines Reagens bestimmt wird, welches spezifisch an das Protein, Polypeptid oder Proteinfragment bindet.

7. Verfahren nach Anspruch 6, wobei das Reagens aus der Gruppe bestehend aus einem Antikörper, Antikörperderivat und Antikörperfragment ausgewählt ist.

8. Verfahren nach einem der Ansprüche 1 bis 4, wobei das Expressionsniveau des Markergens in der Probe durch Bestimmung der Anwesenheit eines transkribierten Polynucleotids in der Probe nachgewiesen wird, welches durch jedes der Markergene kodiert wird, oder einem Teil des transkribierten Polynukleotids.

9. Verfahren nach Anspruch 8, wobei das transkribierte Polynukleotid eine cDNA, mRNA oder hnRNA ist.

10. Verfahren nach einem der Ansprüche 8 bis 9, wobei der Bestimmungsschritt weiter das Amplifizieren des transkribierten Polynukleotids umfasst.

11. Verfahren nach einem der Ansprüche 1 bis 4, wobei das Expressionsniveau des Markergens in den Proben durch Bestimmung der Anwesenheit eines transkribierten Polynukleotids in den Proben nachgewiesen wird, welches unter stringenten Hybridisierungsbedingungen an jedes der Markergene bindet oder an einen Teil der transkribierten Polynukleotide bindet.

12. Verwendung eines Sets zum Nachweis ob ein Patient ein Risiko der Progression von Brustkrebs aufweist, wobei das Set Reagenzien zum Nachweis der Expression eines oder mehrerer Markergene umfasst, und die Markergene die Gruppe bestehend aus den Genen mit einer Nukleinsäuresequenz gemäß SEQ ID NO: 1, 2, 3 und 4 umfassen.

## Revendications

1. Procédé de détermination du fait qu'un échantillon d'un patient contenant des cellules de cancer du sein humaines possède ou non un potentiel de progression tumorale, le procédé comprenant la comparaison :
a) du niveau d'expression des gènes marqueurs dans l'échantillon du patient, et
b) du niveau normal d'expression desdits gènes marqueurs dans un échantillon d'un sujet témoin ne souffrant pas de cancer du sein ou du niveau normal d'expression desdits gènes marqueurs dans un échantillon contenant des cellules d'une lignée de cellules épithéliales mammaires humaines,
lesdits gènes marqueurs comprenant le groupe de gènes marqueurs constitué par les gènes ayant une séquence d'acide nucléique selon SEQ ID NO : 1, 2, 3 et 4 et une différence significative entre le niveau d'expression desdits gènes marqueurs dans l'échantillon du patient et le niveau normal desdits gènes marqueurs dans l'échantillon d'un sujet témoin ne souffrant pas du cancer du sein ou dans l'échantillon contenant des cellules d'une lignée de cellules épithéliales mammaires humaines indique que le patient présente un potentiel de progression tumorale.

2. Procédé selon la revendication 1, dans lequel ladite différence significative comprend une différence d'au moins deux fois entre le niveau d'expression desdits gènes marqueurs dans l'échantillon du patient et le niveau normal d'expression des mêmes gènes marqueurs dans l'échantillon du sujet témoin ou dans l'échantillon contenant des cellules d'une lignée de cellules épithéliales mammaires humaines.

3. Procédé selon la revendication 1, dans lequel ladite différence significative comprend une différence d'au moins cinq fois entre le niveau d'expression desdits gènes marqueurs dans l'échantillon du patient et le niveau normal d'expression des mêmes gènes marqueurs dans l'échantillon du sujet témoin ou dans l'échantillon contenant des cellules d'une lignée de cellules épithéliales mammaires humaines.

4. Procédé selon l'une quelconque des revendications 1 à 3, dans lequel l'échantillon est un échantillon de tissu mammaire ou un fluide associé au sein.

5. Procédé selon l'une quelconque des revendications 1 à 4, dans lequel le niveau d'expression desdits gènes marqueurs dans l'échantillon est évalué par détection de la présence dans l'échantillon d'une protéine codée par chacun desdits gènes marqueurs ou d'un fragment de polypeptide ou de protéine comprenant ladite protéine.

6. Procédé selon la revendication 5, dans lequel la présence de ladite protéine, dudit fragment de polypeptide ou dudit fragment de protéine est détectée en utilisant un réactif qui se lie spécifiquement à ladite protéine, audit fragment de polypeptide ou audit fragment de protéine.

7. Procédé selon la revendication 6, dans lequel le réactif est choisi dans le groupe constitué par un anticorps, un dérivé d'anticorps, et un fragment d'anticorps.

8. Procédé selon l'une quelconque des revendications 1 à 4, dans lequel le niveau d'expression desdits gènes marqueurs dans l'échantillon est évalué par détection de la présence dans l'échantillon d'un polynucléotide transcrit codé par chacun desdits gènes marqueurs ou d'une partie dudit polynucléotide transcrit.

9. Procédé selon la revendication 8, dans lequel le polynucléotide transcrit est un ADNc, un ARNm ou un ARNhn.

10. Procédé selon l'une quelconque des revendications 8 à 9, dans lequel l'étape de détection comprend en outre l'amplification du polynucléotide transcrit.

11. Procédé selon l'une quelconque des revendications 1 à 4, dans lequel le niveau d'expression desdits gènes marqueurs dans les échantillons est évalué par détection de la présence dans les échantillons d'un polynucléotide transcrit qui s'annelle à chacun desdits gènes marqueurs ou qui s'annelle à une partie dudit polynucléotide transcrit, dans des conditions d'hybridation stringentes.

12. Utilisation d'un kit pour évaluer si un patient présente ou non un risque de progression du cancer du sein, le kit comprenant des réactifs pour évaluer l'expression d'un ou de plusieurs gènes marqueurs, et lesdits gènes marqueurs comprenant le groupe constitué par les gènes ayant une séquence d'acide nucléique selon SEQ ID NO : 1, 2, 3 et 4.
